(19) Europäisches Patentamt
European Patent Office
Office européen des brevets

(11) Publication number: **0 122 203**
**B1**

(12) # EUROPEAN PATENT SPECIFICATION

(45) Date of publication of patent specification: **30.12.86**

(51) Int. Cl.⁴: **C 07 C149/36**, C 09 K 15/14, C 10 M105/72

(21) Application number: **84400716.1**

(22) Date of filing: **11.04.84**

(54) **(Hydrocarbylthio)phenols and their preparation.**

(30) Priority: **12.04.83 US 484338**
**14.11.83 US 551336**

(43) Date of publication of application:
**17.10.84 Bulletin 84/42**

(45) Publication of the grant of the patent:
**30.12.86 Bulletin 86/52**

(84) Designated contracting states:
**AT BE CH DE FR GB IT LI LU NL SE**

(56) References cited:
**FR-A- 2 259 131**
**US-A- 3 231 623**
**US-A- 3 246 040**
**US-A- 3 655 773**
**SYNTHESIS, February 1984, pages 117-119, Stuttgart, DE; P.F. RANKEN et al.: "Alkylthiolation of phenols"**

(73) Proprietor: **ETHYL CORPORATION**
**Ethyl Tower 451 Florida Boulevard**
**Baton Rouge Louisiana 70801 (US)**

(72) Inventor: **McKinnie, Bonnie Gary**
**1209 East University Street**
**Magnolia, AR 71753 (US)**
Inventor: **Ranken, Paul Frederick**
**10345 Westwood Avenue**
**Baton Rouge, LA 70809 (US)**

(74) Representative: **Rinuy, Santarelli**
**14, avenue de la Grande Armée**
**F-75017 Paris (FR)**

**EP 0 122 203 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

This invention relates to novel and useful (hydrocarbylthio) phenols containing at least two hydrocarbylthio groups and to a process for their preparation.

Ortho-(hydrocarbylthio) phenols are useful compounds, e. g., as intermediates in the preparation of hypotensive drugs and agricultural chemicals such as plant protection agents, herbicides, pesticides and the like. U. S. Patent 2,923,743 describes a process for the production of aryl-alkyl thioethers by reacting a dialkyl disulfide with aromatic compounds such as phenol, chlorophenol, p-cresol, 2-naphthol, etc., in the presence of suitable condensation agents, such as for example, aluminum chloride, aluminum bromide, ferric chloride, zinc chloride, tin tetrachloride, antimony pentachloride, boron fluoride and bleaching earth. At Column 1, lines 31-36, that patent discloses :

« These condensation agents can be added in different amounts. In general there should be added at least molecular amounts referred to the dialkyl disulfide but there can be used also higher amounts e. g. a 3-fold surplus of the condensation agent. »

U. S. Patent 3,246,040 discloses the synthesis of certain substituted phenolic compounds containing two alkylthio groups and from 0 to 3 halo or nitro groups. This patent discloses that the compounds are useful as pesticides ; as inhibitors of the germination of fungus spores, they are effective fungistats. They are also useful as intermediates in the preparation of biologically active materials such as organic phosphates.

In our U. S. Patent 4,324,920, we disclose a process for the preparation of ortho-(hydrocarbylthio)-phenols by contacting phenols, having at least one hydrogen on a carbon atom ortho to a hydroxy group, with hydrocarbyl disulfides in the presence of catalytic amounts of aluminum phenoxide.

It has been found that hydrocarbylthio-substituted phenols containing either at least two hydrocarbylthio groups on a phenolic ring or hydrocarbylthio groups substituted in either or both meta positions relative to the hydroxy group can be prepared in good yields by contacting phenols, having at least two positions available for substitution on a phenolic ring including either or both of the meta positions, with an excess of hydrocarbyl disulfides in the presence of catalytic amounts of aluminum phenoxide. An excess of hydrocarbyl disulfide is defined to be a quantity of hydrocarbyl disulfide greater than the stoichiometric amount required for the number of hydrocarbylthio groups desired in the (hydrocarbylthio) phenol. A catalytic amount of aluminum phenoxide means less than molecular or equivalent amounts of aluminum phenoxide. The process of this invention requires no solvent and makes use of cheap and available materials. Still further advantages will be apparent from the following disclosure.

It has also been found pursuant to this invention that novel and useful products can be produced by the foregoing process. Among the preferred compounds of the invention are compounds of Formula I :

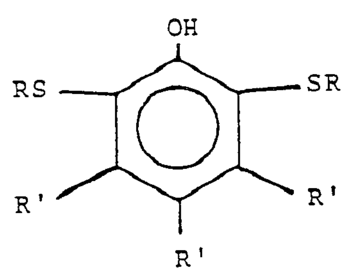

(I)

In Formula I, at least two of the R′ groups are the same or different hydrocarbyl groups devoid of olefinic and acetylenic unsaturation, the remaining R′ group is hydrogen or a hydrocarbyl group devoid of olefinic and acetylenic unsaturation and R is a hydrocarbyl group having up to 10 carbon atoms.

A further group of preferred compounds of this invention are compounds of Formula II :

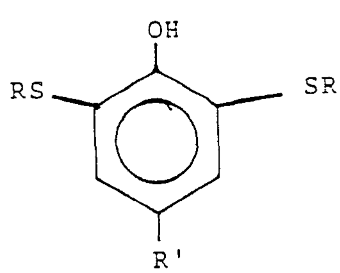

(II)

The R′ group in Formula II is hydrogen or, more preferably, a hydrocarbyl group devoid of olefinic and acetylenic unsaturation, and R is a hydrocarbyl group having up to 10 carbon atoms.

Another preferred group of compounds of this invention is depicted by Formula III :

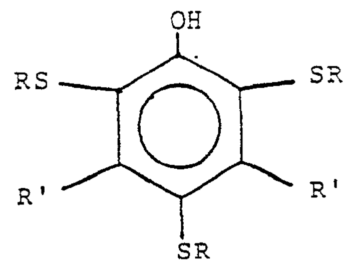

(III)

The R′ groups in Formula III may be the same or different, and are hydrogen atoms or hydrocarbyl groups devoid of olefinic and acetylenic unsaturation and R is a hydrocarbyl group having up to 10 carbon atoms.

Still another group of preferred compounds of this invention are compounds of Formula IV :

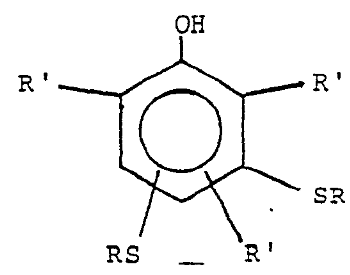

(IV)

In Formula IV, the R′ groups in the 2- and 6-positions are the same or different hydrocarbyl groups devoid of olefinic and acetylenic unsaturation while the other R′ group is a hydrogen atom or a hydrocarbyl group devoid of olefinic and acetylenic unsaturation, which hydrocarbyl group can be the same as or different from the hydrocarbyl groups in the 2- and 6-positions and R is a hydrocarbyl group having up to 10 carbon atoms. As indicated by Formula IV, either the 4- or 5-position is occupied by an -SR group and the other of these positions is occupied by an R′ group.

Particularly preferred compounds within the scope of Formula IV are the compounds of Formula V :

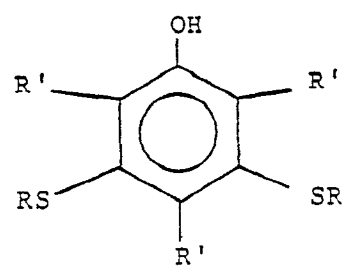

(V)

In Formula V, the R′ groups in the 2- and 6-positions are the same or different hydrocarbyl groups while the R′ group in the 4-position is a hydrogen atom or a hydrocarbyl group, which hydrocarbyl group can be the same as or different from the hydrocarbyl groups in the 2- and 6-positions.

Yet another class of preferred compounds of this invention are those of Formula VI :

(See formula VI p. 4)

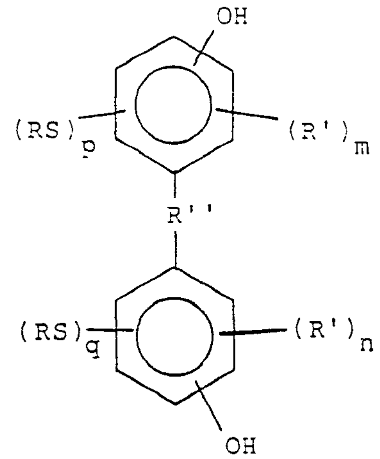

(VI)

The R' groups in Formula VI may be the same or different, and are hydrogen atoms or hydrocarbyl groups devoid of olefinic and acetylenic unsaturation ; R" is an alkylidene or an alkylene group of from 1 to 10 carbon atoms ; m and n are integers from 0 to 4 (both inclusive), p and q are integers from 0 to 4 (both inclusive) and either or both of p and q is an integer of at least 2, the total of m and p and the total of n and q each being 4 ; and R is a hydrocarbyl group containing up to 10 carbon atoms.

A particularly preferred subgroup of compounds within the ambit of Formula VI are compounds of Formula VII :

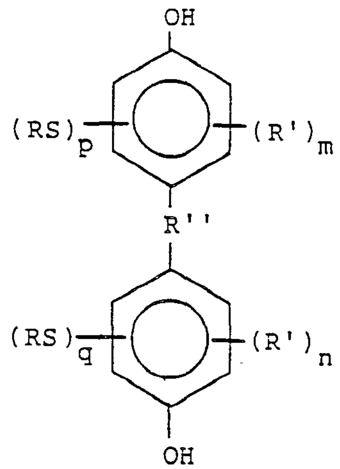

(VII)

The R' groups in Formula VII may be the same or different, and are hydrogen atoms or hydrocarbyl groups.

Still further compounds of the invention are represented by Formula VIII.

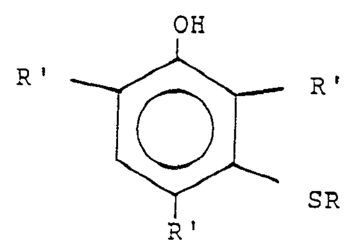

(VIII)

wherein the R' groups are the same or different hydrocarbyl groups and R is a hydrocarbyl group having up to 10 carbon atoms.

Additional compounds of the invention are represented by Formula IX :

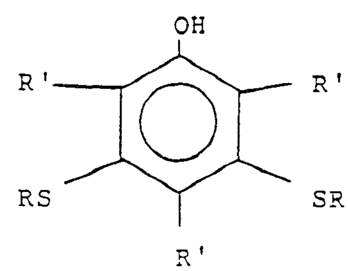

(IX)

wherein the R′ groups are the same or different hydrocarbyl groups and R is a hydrocarbyl group having up to 10 carbon atoms.

Further compounds of the invention are represented by Formula X :

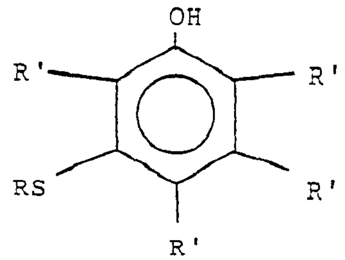

(X)

wherein the R′ groups are the same or different hydrocarbyl groups and R is a hydrocarbyl group having up to 10 carbon atoms.

In the above respective Formulas (I-X), the R groups are hydrocarbyl groups which may differ but which preferably are the same in any given compound. R may be, for example, an aryl group, an alkyl group or a cycloalkyl group typically having up to about 10 carbon atoms, such as phenyl, methyl, ethyl, propyl, n-butyl, sec-butyl, tert-butyl, hexyl, octyl, decyl, cyclopropyl, cyclopentyl, cyclohexyl, cycloheptyl, cyclooctyl, cyclopropylcarbinyl, and the like. Preferably R is an alkyl group containing up to about 4 carbon atoms, most preferably methyl or ethyl.

The R′ hydrocarbyl groups of Formulas I-X are either saturated aliphatic or cycloaliphatic groups or are aromatic groups. In most cases they will be : alkyl of 1 to 50 carbon atoms ; cycloalkyl of from 3 to 20 carbon atoms ; aryl of from 6 to 12 carbon atoms ; alkaryl of from 7 to 20 carbon atoms ; and aralkyl of from 7 to 20 carbon atoms. However, in Formula II the alkyl group may contain as many as 700 or more carbon atoms.

R″ in Formulas VI and VII is an alkylidene or an alkylene group of from 1 to 10 carbon atoms. Alternatively, R″ is a direct carbon-to-carbon linkage connecting the two phenolic rings together. Other linking groups, R″, include an oxygen atom, a sulfur atom, and such groups as

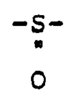 and 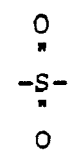 and $-CH_2-S-CH_2-$

and the like.

In Formulas VI and VII, m and n are integers from 0 to 4 (both inclusive), p and q are integers from 0 to 4 (both inclusive) and either or both of p and q is an integer of at least 2. The total of m and p and the total of n and q are each 4.

Another aspect of this invention involves the discovery that the compounds of this invention are effective antioxidants. Tests have shown that various compounds of this invention are highly effective as antioxidants in lubricating oil and these results indicate that the compounds of this invention will exhibit antioxidant properties in a variety of substrates normally susceptible to oxidative deterioration on prolonged exposure to air or oxygen.

Thus, in another of its embodiments this invention further provides an organic material or substrate that is normally susceptible to oxidative deterioration, which contains a (hydrocarbylthio) phenol having either at least two hydrocarbylthio groups on a phenolic ring or a hydrocarbylthio group substituted on either or both meta positions relative to the hydroxy group, such (hydrocarbylthio) phenol being present in a small amount sufficient to inhibit such deterioration.

## Process

In accordance with one aspect of this invention there is provided a process for the preparation of hydrocarbylthiosubstituted phenols containing either at least two hydrocarbylthio groups on a phenolic ring or having a hydrocarbylthio substituent in either or both of the meta positions relative to the hydroxy group which comprises contacting (i. e., reacting) phenols, having either at least two positions or one or both meta positions available for substitution on a phenolic ring, with an excess of a hydrocarbyl disulfide in the presence of an aluminum phenoxide catalyst, the molar ratio of the catalyst to the disulfide being less than 1, preferably between 0.002 and 0.3 and most preferably between 0.01 and 0.1. The process is normally conducted at a temperature within the range of from 0 °C to 300 °C at which the desired reaction takes place. Preferably the process is performed at a temperature of from 100 °C to 300 °C and

most preferably from 100 °C to 200 °C. With the process of this invention one mole of hydrocarbylthiol (RSH) is formed as by-product for each hydrocarbylthio group (RS-) substituted onto the phenol. Without being bound to a particular mechanism or theory, it appears that the reacting hydrocarbyl disulfide is split into two species or moieties, one being substituted onto the phenol the other forming the corresponding hydrocarbylthiol by-product. In a particularly preferred embodiment, the process of this invention is conducted in such a way that the hydrocarbylthiol by-product is removed from the reaction vessel essentially as rapidly as it is formed. This can be accomplished for example by conducting the reaction at reflux temperature and/or under reduced pressures (i. e. below atmospheric pressure) and/or under a sweep of an inert gas so that the hydrocarbylthiol by-product evolved is rapidly removed or purged from the reaction vessel. Alternatively, the reaction may be carried out at pressures greater than one atmosphere with the hydrocarbylthiol by-product being removed by distillation.

In still another preferred embodiment of this invention the hydrocarbyl disulfide reactant is a saturated aliphatic or cycloaliphatic hydrocarbyl disulfide, most preferably a lower alkyl disulfide.

The aluminum phenoxide catalyst used in the practice of this invention can be formed in various ways. For example it can be formed by contacting aluminum metal, preferably in the form of turnings, powders, particles and the like, with the phenol at elevated temperatures (e. g. 100-200 °C) until cessation of hydrogen evolution. Another way of forming the catalyst used in this invention is by simply contacting an aluminum alkyl such as triethylaluminum with the phenol. Still another method of forming the catalyst used herein involves contacting aluminum chloride (i. e. $AlCl_3$) and the phenol at elevated temperatures, e. g. between 100 and 200 °C, and purging the system of the hydrogen chloride generated during the preparation of the phenoxide catalyst. The source of the aluminum phenoxide catalyst does not materially affect the reaction and other methods known in the art for the formation of these compounds can be successfully employed herein. Exemplary methods for preparing aluminum phenoxide catalysts useful in the process of this invention appear in U. S. Patents 2,831,898, 2,923,745 and 3,200,157. The preferred catalyst is aluminum triphenoxide, although a diphenoxide catalyst, e. g., diphenoxyaluminum chloride (which may be formed in the reaction of aluminum chloride and phenol) or diphenoxyaluminum hydroxide (which may be formed by reaction of aluminum chloride and phenol containing small amounts of water), can also be used in the practice of this invention.

Hydrocarbyl disulfides which may be employed in the practice of this invention include the alkyl disulfides and the cycloalkyl disulfides, these respective disulfides preferably having up to 10 carbon atoms in each aryl, alkyl or cycloalkyl group. Preferably the hydrocarbyl disulfide is a lower alkyl disulfide, i. e., an alkyl disulfide in which each alkyl group contains 1 to 6 carbon atoms, such as, for example, methyl disulfide and ethyl disulfide, these two compounds being particularly preferred reactants because of their availability and good reactivity in the process. Aromatic disulfides, unsaturated aliphatic disulfides, unsaturated cycloaliphatic disulfides, as well as substituted hydrocarbyl disulfides, may be used provided they do not interfere with the reaction. However, if the final product is to be used as an antioxidant in lubricating oils, it is preferred that the product be essentially halogen-free. Hydrocarbyl disulfides which may be used herein include, for example, phenyl disulfide, methyl disulfide, ethyl disulfide, methyl ethyl disulfide, n-butyl disulfide, sec-butyl disulfide, tert-butyl disulfide, propyl disulfide, cyclopentyl disulfide, cyclohexyl disulfide, cycloheptyl disulfide and the like. When it is desired to introduce two different -SR groups into the product, two different hydrocarbyl disulfides will normally be employed in the reaction, either simultaneously or consecutively. The total quantity of the two disulfides used will be in excess of the stoichiometric amount required to introduce the number of -SR groups desired in the product.

Phenols used herein can be mono-or poly-nuclear, e. g. the naphthols. The phenols can contain substituents as long as they do not interfere with the reaction, and as long as there are at least two positions available for substitution on a phenolic ring by hydrocarbylthio groups or the phenols are 2,4,6-tri- or 2,3,4,6-tetra-substituted phenols. The aryl portion of the hydroxy aromatic compound may be linked to or fused with other cyclic systems including heterocyclic systems such as those containing oxygen, nitrogen and/or sulfur atoms in the rings. Examples of the aryl portion of the hydroxy aromatic compound include but are not limited to the following : benzene, biphenyl, 2,2-diphenylpropane, 1,3-dimethylbenzene, 1,2,3-trimethylbenzene, naphthalene, anthracene, phenanthrene, indene, benzofuran, thionaphthene, 1,2-benzopyran, 1,4-benzopyran, quinoline, isoquinoline, acenaphthene, fluorene, dibenzopyrrole, xanthene, thianthrene, naphthacene, chrysene, pyrene, triphenylene, biphenyl, and the like. In the phenolic derivatives of these compounds the hydroxy group is of course bonded to a nuclear carbon atom of a benzene ring.

Substituents which can be present in the hydroxy aromatic compounds should be relatively inert under the conditions of the reaction. For example, any of the previously mentioned aromatics may be substituted in a variety of positions with alkyl, aryl, aralkyl, alkaryl and cycloalkyl radicals, chlorine, fluorine, alkoxy, and the like. Desirably, the phenolic reactant does not contain bromine or iodine substituents as these appear to interfere with the desired reaction. If the final product is to be used as an antioxidant in lubricating oils, it is preferred that the product be formed from a phenolic compound that is essentially halogen-free. A few representative examples of these substituted phenols that may be used in the process of this invention include but are not limited to : 4-chlorophenol, 4-(4-chlorophenyl)phenol, p-cresol, 4-phenylphenol, 4-benzylphenol, 4-phenoxyphenol, and the like.

Those skilled in the art will appreciate that factors of steric hindrance and the relative position of the substituent(s) on the ring should be taken into account when determining what substituents may be present in the starting phenolic compound. Also, since some tertiary aliphatic groups ortho to an aromatic hydroxy group may undergo dealkylation, aliphatic groups in the ortho position(s) are preferably secondary aliphatic groups, and most preferably are primary aliphatic groups.

A preferred class of phenolic reactants are those which can be represented by Formula XI :

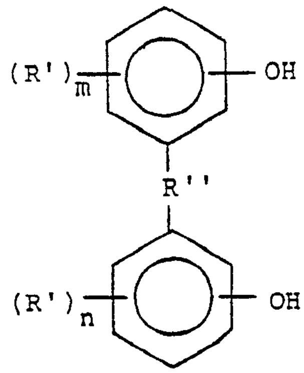

(XI)

Another preferred class of phenolic reactants for use in the process are those which can be presented by Formula XII :

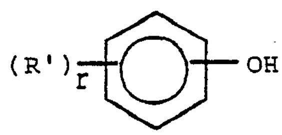

(XII)

Another class of phenolic reactants for use in the process are those which can be presented by Formula XIII :

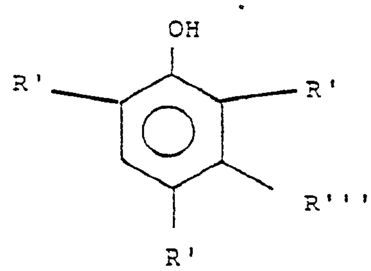

(XIII)

In Formulas XI, XII and XIII, the R′ groups are the same or different hydrocarbyl groups and R‴ is hydrogen or the same or different hydrocarbyl groups. Typically, each R′ hydrocarbyl group will be devoid of olefinic and acetylenic unsaturation and usually will contain no more than 50 carbon atoms. Thus, the R′ groups may be an alkyl group having from 1 to 50 or more (and preferably from 1 to 20) carbon atoms, such as, for example, methyl, ethyl, propyl, isopropyl, tert-butyl, pentyl, heptyl, eicosyl, triacontyl, pentacontyl and the like ; an aryl group having from 6 to 12 carbon atoms, such as, for example, phenyl, 4-biphenylyl, 1-naphthyl, and the like ; an aralkyl group having from 7 to 20 carbon atoms, such as, for example, benzyl, phenylethyl (phenethyl), phenylhexyl, phenylheptyl, phenyloctyl, phenylnonyl, phenylundecyl, phenyldodecyl, phenyltridecyl, phenyltetradecyl, phenylcyclopropyl, phenylcyclohexyl, 2,4-dimethylbenzyl, and the like ; an alkaryl group having from 7 to 20 carbon atoms, such as, for example, tolyl, xylyl, mesityl, butylphenyl, iso-butylphenyl, sec-butylphenyl, tert-butylphenyl, ethylphenyl, pentylphenyl, 4-methylbiphenyl, 3-methylbiphenyl and the like ; and a cycloalkyl group having from 3 to 20 carbon atoms, such as, for example, cyclopropyl, cyclohexyl, cyclooctyl, bicyclohexyl, butylcyclohexyl, methylcyclohexyl, cyclobutyl, cyclodecyl, cyclododecyl, cyclohexylphenyl, cyclopentyl, and the like, as well as other hydrocarbyl groups exemplified by cyclopropylphenyl, α-cyclopropylphenylmethyl and the like. However, long chain monoalkyl phenols (usually the 4-alkyl isomer) made by alkylating phenol with propylene oligomers or isobutylene oligomers of molecular weights as high as 10 000 may be used. Of these long chain monoalkyl phenols, 4-alkylphenols in which the alkyl group has from 50 to 700 carbon atoms are preferred.

In Formula XI, m and n are integers of from 0 to 4 (both inclusive) with the understanding that there

will be at least one phenolic ring with at least two positions available for substitution by hydrocarbylthio groups.

In Formula XI, R" is a suitable divalent linking group such as alkylidene or alkylene.

Preferably R" is an alkylidene or an alkylene group of from 1 to 10 carbon atoms, such as, for example, methylidene, ethylidene, propylidene, isopropylidene, butylidene, hexylidene, decylidene, methylene, ethylene, propylene, isopropylene, butylene, hexylene, decylene, and the like. Preferably the alkylidene or alkylene group has up to about 4 carbon atoms. In the most preferred compounds of Formula XI, R" is either isopropylidene or methylene.

In Formula XII, r is an integer of from 0 to 3, and preferably from 0 to 2 (both inclusive).

Some examples of the phenolic compounds represented by Formulas XI and XII include but are not limited to : phenol, p-cresol, a mixture of o- and p-cresols, m-cresol, a mixture of 2- and 4-isomers of nonylphenol, 4-tert-butylphenol, 4-phenylphenol, 2,2'-diphenol, 4-(1,1,3,3-tetramethylbutyl)phenol, 3,5-dimethylphenol, a mixture of o-, m-, and p-cresols, 4-pentacontylphenol, 2-cyclohexylphenol, 4-cyclooctylphenol, 4-(2,4-dimethylcyclohexyl)phenol, 2,6-dimethylphenol, 2,5-dimethylphenol, 3,4-dimethylphenol, 3,4,5-trimethylphenol, 4-(2-eicosyl)phenol, 4-(4-tridecylbenzyl)phenol, 4,4'-diphenol, 4-(3,5-di-sec-heptylphenyl)phenol, 2-sec-butylphenol, 2-ethylphenol, 2,6-diethylphenol, 2-(α-methylbenzyl)phenol, 2-cyclopentylphenol, a mixture of isomeric o-sec-pentacontylphenols, 4-tert-amylphenol, 4-(α-methyl-4-dodecylbenzyl)phenol, 2-phenylphenol, 2-(4-tetradecylphenyl)phenol, 2-triacontylphenol, 2-isopropylphenol, 2-(α-methyl-4-sec-amylbenzyl)phenol, 4,4'-isopropylidenediphenol (bisphenol A), 4,4'-methylenebisphenol, 2,2'-methylenebisphenol, 4,4'-methylenebis(2-ethylphenol), 4,4'-propylidenebis-phenol, 4,4'-butylidenebis(phenol), 2,2'-ethylidenebisphenol, 4,4'-pentylidenebisphenol, 1-naphthol, 2-naphthol, 2-ethoxyphenol, 3-ethoxyphenol, 4-ethyxyphenol, 4-octyloxyphenol, 4-hexacosylphenol, 4-triacontylphenol, 4-tetracontylphenol, and the like.

Some examples of the phenolic compounds represented by Formula XIII include but are not limited to : 2,4,6-trimethylphenol, 2,4,6-triethylphenol, 2,4,6-tripropylphenol, 2,4,6-tricyclopropylphenol and 2,4,6-trihexylphenol.

A particularly preferred embodiment of this invention involves reaction of (i) a phenol of Formula XIII above wherein the R‴ is group hydrogen and the R' groups are alkyl groups of from 1 to about 4 carbon atoms with (ii) a lower alkyl disulfide, i. e., each alkyl group has from 1 to 6 carbon atoms, using an aluminum phenoxide catalyst in the manner described above. It is especially desirable to perform these embodiments under conditions whereby the alkyl mercaptan by-product is removed from the reaction mixture during the course of the reaction.

A particularly preferred embodiment of this invention involves reaction of (i) a phenol of Formula XI above wherein m and n are both zero and R" is an alkylidene group or alkylene group containing up to 4 carbon atoms with (ii) with (ii) a lower alkyl disulfide, i. e. each alkyl group has from 1 to 6 carbon atoms, using an aluminum phenoxide catalyst in the manner described above. Another particularly preferred embodiment involves conducting this reaction using a phenol of Formula XII above wherein r is an integer from zero to 1 (both inclusive) and R' is a hydrocarbyl group devoid of olefinic and acetylenic unsaturation and containing up to 50 carbon atoms. In a further particularly preferred embodiment, the phenol used is a monoalkylphenol in which the alkyl group has from 50 to 700 or more carbon atoms. Still another particularly preferred embodiment involves conducting this reaction with a phenol of Formula XII above wherein r is 2 and each R' is a hydrocarbyl group devoid of olefinic and acetylenic unsaturation and containing up to 30 carbon atoms. It is especially desirable to perform these embodiments under conditions whereby the alkyl mercaptan by-product is removed from the reaction mixture during the course of the reaction.

Those skilled in the art will recognize that phenolic compounds having one or more of the ortho/para positions already occupied by substituent groups may be utilized in the process of this invention, providing the presence of those substituent groups still permit the phenolic compound to be substituted by at least two hydrocarbylthio groups. Generally speaking, however, it is preferable, in terms of ease of reaction, to use phenolic compounds whereby one or more of the positions available for substitution by hydrocarbylthio groups are selected from amongst the ortho and para positions.

In practicing the process of this invention the hydrocarbyl disulfide is normally added to the phenolaluminum phenoxide mixture although the order of addition is not critical. Once the phenol and hydrocarbyl disulfide have been contacted in the presence of the catalyst the reaction is allowed to proceed at temperatures and times which can vary depending upon such considerations as the boiling point of the hydrocarbylthio generated during reaction, the percent yield of product desired, the melting point of the reactants and the like. In general, when the reaction is carried out at atmospheric pressure, temperatures slightly above the boiling point of the hydrocarbyl disulfide are employed. In a particularly preferred aspect, the mixture is maintained at or near its reflux temperature. Of course if pressures below atmospheric pressure are employed the corresponding temperature may also be decreased. In a preferred embodiment as much of the hydrocarbylthio by-product as is feasible is removed, e. g. by vacuum distillation and the like. Total reaction times of more than one day are typically employed when the reaction is run at atmospheric pressure. However, if desired, the reaction may be conducted at pressures greater than atmospheric pressure with provision being made for removal of the alkyl mercaptan by-product and thereby shorter reaction times may be employed.

In practicing this invention a broad range of proportions of reactants can be utilized. That is, the relative molar amounts of phenol to the disulfide can vary widely ; however, the relative proportions employed normally fall within the range of from 1 : 2.1 to 1 : 20, and most preferably within the range of from 1 : 2.5 to 1 : 6. Furthermore, while the process of this invention can be carried out without the use of solvents or diluents they may nevertheless be used, if desired, so long as they are inert to the reaction disclosed herein. Hydrocarbons and ethers are illustrative of the solvents that may be employed. The process is preferably carried out under substantially anhydrous conditions, but conditions wherein trace or small amounts of water are present can be used. Accordingly, an inert atmosphere is normally employed in the present process.

Compounds

As noted above, another aspect of this invention involves the provision of novel and useful compounds producible by the process described above. These compounds are depicted hereinabove in Formulas I through X.

Illustrative examples of compounds which are provided in accordance with this invention include but are not limited to such compounds as the following :

Formula I

3,5-dimethyl-2,6-bis(methylthio)phenol,
3,4,5-trimethyl-2,6-bis(methylthio)phenol,
3,4-dimethyl-2,6-bis(methylthio)phenol,
3,5-dimethyl-2,6-bis(ethylthio)phenol,
3,4-dimethyl-2,6-bis(propylthio)phenol,
3-ethyl-5-methyl-2,6-bis(methylthio)phenol, and
3,5-diethyl-2,6-bis(butylthio)phenol.

Formula II

2,6-bis(methylthio)phenol,
2,6-bis(ethylthio)phenol,
2,6-bis(nonylthio)phenol,
2,6-bis(cyclopentylthio)phenol,
4-methyl-2,6-bis(methylthio)phenol,
4-methyl-2,6-bis(butylthio)phenol,
4-methyl-2,6-bis(decylthio)phenol,
4-methyl-2,6-bis(cyclohexylthio)phenol,
4-ethyl-2,6-bis(methylthio)phenol,
4-butyl-2,6-bis(ethylthio)phenol,
4-tert-butyl-2,6-bis(methylthio)phenol,
4-tert-butyl-2,6-bis(propylthio)phenol,
4-tert-amyl-2,6-bis(methylthio)phenol,
4-(1,1,3,3-tetramethylbutyl)-2,6-bis(methylthio)phenol,
4-(1,1,3,3-tetramethylbutyl)-2,6-bis(ethylthio)phenol,
4-(1,1,3,3-tetramethylbutyl)-2,6-bis(decylthio)phenol,
4-nonyl-2,6-bis(methylthio)phenol,
4-eicosyl-2,6-bis(ethylthio)phenol,
4-pentacontyl-2,6-bis(methylthio)phenol,
4-cyclohexyl-2,6-bis(methylthio)phenol,
4-phenyl-2,6-bis(methylthio)phenol,
4-(p-tolyl)-2,6-bis(ethylthio)phenol, and
4-biphenylyl-2,6-bis(methylthio)phenol.

Formula III

2,4,6-tris(methylthio)phenol,
2,4,6-tris(ethylthio)phenol,
2,4,6-tris(propylthio)phenol,
2,4,6-tris(decylthio)phenol,
3-methyl-2,4,6-tris(methylthio)phenol,
3-methyl-2,4,6-tris(ethylthio)phenol,
3,5-dimethyl-2,4,6-tris(methylthio)phenol,
3,5-dimethyl-2,4,6-tris(ethylthio)phenol, and
3-ethyl-5-methyl-2,4,6-tris(methylthio)phenol.

Formula IV

2,6-dimethyl-3,4-bis(methylthio)phenol,
2,6-dimethyl-3,4-bis(ethylthio)phenol,
2,6-dimethyl-3,4-bis(octylthio)phenol,
2,6-diethyl-3,4-bis(methylthio)phenol, and
2-ethyl-6-isopropyl-3,4-bis(methylthio)phenol.

Formula V

2,6-dimethyl-3,5-bis(methylthio)phenol,
2,6-dimethyl-3,5-bis(ethylthio)phenol,
2-ethyl-6-methyl-3,5-bis(methylthio)phenol,
2,6-diethyl-3,5-bis(methylthio)phenol,
2,4,6-trimethyl-3,5-bis(methylthio)phenol,
2,4,6-trimethyl-3,5-bis(ethylthio)phenol, and
2-ethyl-4,6-dimethyl-3,5-bis(methylthio)phenol.

Formula VI

2,2'-methylenebis[4,6-bis(methylthio)phenol],
2,2'-methylenebis[4,6-bis(hexylthio)phenol],
4,4',6,6'-tetra(methylthio)-2,2'-ethylidenediphenol,
4,4',6-tris(methylthio)-2,2'-isopropylidinediphenol,
4,4',6,6'-tetra(ethylthio)-2,2'-butylidenediphenol.
4,4',6-tris(ethylthio)-2,2'-butylidenediphenol,
2',4,6,6'-tetra(methylthio)-2,4'-diphenol,
2,2'-bis[4,6-bis(methylthio)phenol], and
2,2'-bis[4,6-bis(ethylthio)phenol].

Formula VII

2,2',6,6'-tetra(methylthio)-4,4'-isopropylidenediphenol,
2,2',6,6'-tetra(ethylthio)-4,4'-isopropylidenediphenol,
2,2',6,6'-tetra(propylthio)-4,4'-isopropylidenediphenol,
2,2',6,6'-tetra(butylthio)-4,4'-isopropylidenediphenol,
3,3'-dimethyl-2,2',6,6'-tetra(methylthio)-4,4'-isopropylidenediphenol,
3,3'-diethyl-2,2',6,6'-tetra(ethylthio)-4,4'-isopropylidenediphenol,
2,2',6-tris(methylthio)-4,4'-isopropylidenediphenol,
2,2',6-tris(ethylthio)-4,4'-isopropylidenediphenol,
2,2',6-tris(propylthio)-4,4'-isopropylidenediphenol,
2,2',6-tris(butylthio)-4,4'-isopropylidenediphenol,
3,3'-dimethyl-2,2',6-tris(methylthio)-4,4'-isopropylidenediphenol,
3,3'-diethyl-2,2',6-tris(ethylthio)-4,4'-isopropylidenediphenol,
4,4'-methylenebis[2,6-bis(methylthio)phenol],
4,4'-methylenebis[2,6-bis(ethylthio)phenol],
4,4'-methylenebis[2,6-bis(propylthio)phenol],
4,4'-methylenebis[2,6-bis(butylthio)phenol],
2,2',6,6'-tetra(methylthio)-4,4'-ethylidenediphenol,
2,2',6,6'-tetra(ethylthio)-4,4'-ethylidenediphenol,
2,2',6,6'-tetra(methylthio)diphenol,
2,2',6,6'-tetra(ethylthio)diphenol,
2,2',6,6'-tetra(propylthio)diphenol,
2,2',6,6'-tetra(butylthio)diphenol,
2,2',6-tris(methylthio)diphenol,
2,2',6-tris(ethylthio)diphenol,
3,3'-dimethyl-2,2',6,6'-tetra(propylthio)diphenol,
3,3',5-tris(methylthio)-4,4'-dihydroxydiphenylether, and
3,3',5,5'-tetra(methylthio)-4,4'-dihydroxydiphenylether.

Formula VIII

2,4,6-trimethyl-3-(methylthio)phenol,
2,4,6-trimethyl-3-(ethylthio)phenol,

2,4,6-trimethyl-3-(propylthio)phenol,
2,4,6-trimethyl-3-(butylthio)phenol,
2,4,6-triethyl-3-(methylthio)phenol,
2,4,6-triethyl-3-(ethylthio)phenol,
2,4,6-triethyl-3-(propylthio)phenol,
2,4,6-triethyl-3-(butylthio)phenol,
2,4,6-tributyl-3-(methylthio)phenol,
2,4,6-tributyl-3-(ethylthio)phenol,
2,4,6-tributyl-3-(propylthio)phenol,
2,4,6-tributyl-3-(butylthio)phenol, and
2,6-dimethyl-4-sec-eicosyl-3-(methylthio)phenol.

Formula IX

2,4,6-trimethyl-3,5-bis(methylthio)phenol,
2,4,6-trimethyl-3,5-bis(ethylthio)phenol,
2,4,6-triethyl-3,5-bis(methylthio)phenol,
2,4,6-triethyl-3,5-bis(ethylthio)phenol,
2,4,6-triethyl-3,5-bis(propylthio)phenol,
2,4,6-tripropyl-3,5-bis(methylthio)phenol,
2,4,6-tributyl-3,5-bis(methylthio)phenol, and
2,6-dimethyl-4-n-octadecyl-3,5-bis(methylthio)phenol.

Formula X

2,3,4,6-tetramethyl-5-(methylthio)phenol, and
2,3,4,6-tetramethyl-5-(ethylthio)phenol.

Still other compounds which may be produced by the process described hereinabove include such compounds as :
6-nonyl-2,4-bis(methylthio)phenol,
2,4-bis(ethylthio)phenol,
3,5-dimethyl-2,4-bis(methylthio)phenol
2-methyl-4,6-bis(ethylthio)phenol,
6-methyl-2,4-bis(methylthio)phenol,
2,4-bis(butylthio)phenol,
3,5-dimethyl-2,4-bis(ethylthio)phenol, and the like.

Of interest is the production by the process of this invention of compounds of Formulas IV and V wherein the phenolic compound is substituted by a hydrocarbylthio group in either or both of the meta positions relative to the hydroxyl group. Reference to processes giving direct meta-substitution of phenols is rarely encountered in the scientific literature, especially where the para position carries a hydrogen atom.

The preparation of the compounds of this invention will be still further apparent from the following illustrative examples which are not intended to limit the invention in any manner.

In Examples 1-10 reactions were carried out under nitrogen in a 3-neck round bottom flask equipped with a magnetic stirring bar, thermometer, and a Vigreux column. On top of the Vigreux column was a reflux condenser.

Also, unless noted otherwise, distillations were performed using a 7-cm Vigreux column except in Example 11 which used a 15 cm (6-inch) Vigreux column.

Further, VPC analyses were performed on either a 4.6 m (15 foot) OV-25 (8 %) or a 3 m (10 foot) OV-101 (6 %) column programmed from 100 °C to 280 °C at 10 °C per minute, unless noted otherwise.

Example 1

Preparation of 4-Methyl-2,6-bis(methylthio)phenol

A mixture of 10.8 g (0.10 mole) of p-cresol and 10 ml of cyclohexane was distilled free of cyclohexane to remove water. Aluminum (about 10-20 mesh), 0.135 g (5.0 mmole), was then added to the p-cresol and the mixture was heated at 150-170 °C until all the metal dissolved. The mixture was cooled and 10 ml of xylene and 22.5 ml (0.25 mole) of methyl disulfide were added. This mixture was refluxed.

After a total of 6 days at reflux the mixture was diluted with THF, extracted with 6N HCl and then saturated NaCl, and dried over $Na_2SO_4$. Distillation gave 13.06 g (65 % yield) of a product which was a clear liquid having a b.p. of 108-111 °C at 0.1 mm. NMR analysis showed the product to be 4-methyl-2,6-bis(methylthio)phenol.

Example 2

Preparation of 4-Tert-butyl-2,6-bis(methylthio)phenol

A mixture of 30.0 g (0.20 mole) of 4-tert-butylphenol and 0.34 g (0.013 mole) of aluminum (10-20 mesh) was heated to 190 °C. No reaction was observed so the mixture was cooled and 0.20 ml (0.0015 mole) triethylaluminum was added. Reheating the mixture to 190 °C led to gas evolution and the aluminum dissolved. The mixture was cooled to about 100 °C, 40 ml (0.45 mole) of methyl disulfide was added and this mixture was then refluxed for 3 days.

VPC analysis showed 48 area % of methyl disulfide, 14 area % of 4-tert-butylphenol, 35 area % of 4-tert-butyl-2-(methylthio)phenol, and 2.8 area % of 4-tert-butyl-2,6-bis-(methylthio)phenol. Ten additional ml (0.11 mole) of methyl disulfide was added to the mixture and refluxing was continued.

After a total of nine days of refluxing the mixture was stripped of excess methyl disulfide, dissolved in diethyl ether and extracted with 2N HCl. The ether layer was then washed with saturated NaCl and dried over $MgSO_4$. Concentration and distillation gave, after a small forecut, 33.7 g (70 % yield) of a product with a b. p. of 130-131 °C/0.3 mm. NMR analysis showed the product to be 4-tert-butyl-2,6-bis(methylthio)phenol.

Example 3

Preparation of 4-Phenyl-2,6-bis(methylthio)phenol

Triethylaluminum, 1.7 ml (0.012 mole), was very carefully added dropwise to 34.0 g (0.20 mole) of 4-phenylphenol. The mixture was heated to 170 °C, cooled to room temperature and 40 ml (0.45 mole) of methyl disulfide was added. This mixture was refluxed for 24 hours. Then 10 ml (0.11 mole) more of methyl disulfide was added and the refluxing was continued.

After a total of seven days of refluxing the mixture was cooled, stripped of excess methyl disulfide and diluted with 2N HCl. The mixture was then extracted with a $CH_2Cl_2$/THF solution and the organic phase was stripped of solvent leaving a black oil. Simple distillation gave 21.3 g of a product with a b. p. of 190°-207 °C/1.0 mm. This product was recrystallized from $CHCl_3$/heptane giving 19.3 g (37 % yield) of a pale yellow solid having a m. p. of 87-88 °C. Analysis by NMR showed the product to be 4-phenyl-2,6-bis(methylthio)phenol.

Example 4

Preparation of 2,4,6-Tris(methylthio)phenol

Phenol, 14.1 g (0.15 mole), was reacted with 0.30 g (0.011 mole) of aluminum (8-10 mesh) at 160-180 °C until all the aluminum dissolved. The mixture was cooled, 60 ml (0.67 mole) of methyl disulfide was added and this mixture was refluxed for six days. The results of VPC analysis of samples taken after 4 days and after 6 days of refluxing are given in Table I.

Table I

VPC Analysis Results for Example 4

| Compound | Normalized Area % 4 days | Normalized Area % 6 days |
|---|---|---|
| 2-(methylthio)phenol | 3.8 | 0.25 |
| 2,4-bis(methylthio)phenol | 13 | < 1 |
| 2,6-bis(methylthio)phenol | 38 | 39 |
| 2,4,6-tris(methylthio)phenol | 44 | 60 |

The reaction mixture was diluted with diethyl ether, extracted with 6N HCl, washed with saturated NaCl and dried over $Na_2SO_4$. Distillation of the mixture gave 5.8 g (21 % yield) of 2,6-bis(methylthio) phenol having a b. p. of 108-113 °C/0.15 mm and 13.9 g (40 % yield) of 2,4,6-tris(methylthio)phenol having a b. p. of 147-151 °C/0.15 mm. The identity of each was confirmed by NMR analysis.

Example 5

Reaction of Nonylphenol with Methyl Disulfide

A mixture of 39.6 g (0.18 mole) of Eastman practical grade nonylphenol (about a 1 : 1 ratio of the o-/p-isomers as shown by NMR) and 0.34 g (0.013 mole) aluminum was heated up to 260 °C. No reaction was observed to occur and therefore the mixture was cooled and a few drops of triethylaluminum was added. The mixture was reheated to 180 °C which resulted in the aluminum reacting. Methyl disulfide, 20 ml (0.22 mole) was added at 100 °C and the mixture was refluxed for 36 hours. Ten ml (0.11 mole) more of methyl disulfide was added and refluxing was continued.

After refluxing one more day, 20 ml (0.22 mole) more of methyl disulfide was added to the reaction mixture and refluxing was continued an additional 3 days. The reaction mixture was cooled, diluted with THF/pentane, extracted with 6N HCl then with saturated NaCl and dried over $Na_2SO_4$.

Distillation gave a center cut fraction of 32.0 g (57 % yield) having a b. p. of 150-152 °C/0.2 mm. $^1H$ and $^{13}C$ NMR indicated this was a mixture of 4-nonyl-2,6-bis(methylthio)phenol and 6-nonyl-2,4-bis(methylthio)phenol.

## Example 6

Reaction of Phenol with Ethyl Disulfide

Phenol, 10.0 g (0.106 mole), and 0.21 g (7.8 mole) of aluminum were heated in a 170 °C oil bath until all gas evolution ceased and all the aluminum dissolved. The mixture was cooled and 33 ml (0.27 mole) of ethyl disulfide was added. This mixture was heated to reflux.

After 16 hours Sample (A) was withdrawn and analyzed by VPC. After 23 hours Sample (B) was taken and after 41 hours Sample (C) was taken.

The reaction mixture was then stripped of excess ethyl disulfide, taken up in THF/diethyl ether, extracted with 6N HCl and then with saturated NaCl.

Distillation of the mixture yielded four fractions with are described in Table II. Compound identification was confirmed by NMR analysis.

Table II

Distillation results of the Mixture of Example 6

| Fraction | b.p. | Major Component |
|---|---|---|
| (D) | 60-100/0.1 mm | -- |
| (E) | 100-101/0.1 mm | Mixture of 2,4- and 2,6-bis(ethylthio)phenol |
| (F) | 101-146/0.1 mm | 2,6-bis(ethylthio)phenol |
| (G) | 146-154/0.1 mm | 2,4,6-tris(ethylthio)phenol |

The results of VPC analysis of the samples and fractions are given in Table III.

Table III

VPC Analysis of Samples and Fractions of Example 6

| | Area % | | | | | | |
|---|---|---|---|---|---|---|---|
| Compound | (A) | (B) | (C) | (D) | (E) | (F) | (G) |
| Ethyl disulfide | 58.5 | 52.1 | 39.3 | - | - | - | - |
| Phenol | 4.7 | 1.4 | 0.6 | - | - | - | - |
| 2-(ethylthio)-phenol | 22.7 | 24.2 | 17.4 | 4.5 | - | - | - |
| 4-(ethylthio)-phenol | 5.1 | 2.8 | 1.1 | 10 | 3.1 | - | - |
| 2,4-bis(ethyl-thio)phenol | 6.9 | 11.3 | 13.1 | 40 | 43.7 | 22 | - |

(Continued)

| Compound | Area % (A) | (B) | (C) | (D) | (E) | (F) | (G) |
|---|---|---|---|---|---|---|---|
| 2,6-bis(ethylthio)phenol | 2.0 | 5.9 | 18.0 | 39 | 52.8 | 63 | - |
| 2,4,6-tris(ethylthio)phenol | 0.1 | 1.5 | 7.7 | - | - | 11 | 86 |

Example 7

Reaction of 4,4'-Isopropylidenediphenol(Bisphenol A) with Methyl Disulfide

Bisphenol A, 45.6 g (0.20 mole), was dissolved in 20 ml of xylene at 150 °C, cooled to about 100 °C and then cautiously treated with 2.7 ml (0.020 mole) of triethyl-aluminum. This mixture was reheated to 160 °C, cooled to 120 °C and then 100 ml (1.11 mole) of methyl disulfide was added.

After 7 days at reflux the mixture, which was black in color, was cooled, poured onto cold dilute HCl and extracted with diethyl ether. The organic phase was washed with cold dilute NaCl and dried over $Na_2SO_4$. Removal of the solvent left a black oil which could not be crystallized from toluene/pentane.

The oil was dissolved in diethyl ether and extracted with dilute aqueous sodium hydroxide. The aqueous extract was acidified with HCl, extracted with diethyl ether and the etheral extract was dried ($Na_2SO_4$), filtered and the solvent removed to give a black oil. The oil was concentrated by distillation, dissolved in $CH_2Cl_2$ and passed through a column of silica gel. Removal of the solvent gave a dark yellowish brown oil which was distilled to give a viscous yellow oil having a b. p. of 224-255 °C/0.2-0.3 mm. VPC analysis showed the yellow oil to contain 31 area % of 2,2',6-tris(methylthio)-4,4'-isopropylidenediphenol and 60 area % of 2,2',6,6'-tetra(methylthio)-4,4'-isopropylidenediphenol. NMR and gc/ms analysis confirmed the identity of the compounds.

Example 8

Preparation of 3,5-Dimethyl-2,4,6-tris(methylthio)phenol

A mixture of 12.2 g (0.1 mole) of Eastman 3,5-dimethylphenol and 0.16 g (0.006 mole) of aluminum was heated in a 170 °C oil bath. Vigorous gas evolution was observed. After 20-30 minutes no further reaction was observed. The mixture was cooled, 36 ml (0.4 mole) of methyl disulfide was added and the mixture was refluxed for 20 days.

The reaction mixture was cooled and stirred five minutes with 10 ml 1N HCl. The reaction mixture was observed to thicken and then thin. This mixture was then diluted with 20 ml diethyl ether, separated and the aqueous fraction was extracted three times with 20 ml portions of diethyl ether. The organic fractions were combined and the solvent was removed on a rotary evaporator. The dark liquid obtained was distilled through a 6" jacketed Vigreux column at full (0.3 mm) vacuum. Table IV gives the GC analysis results.

Table IV

GC Analysis of the Distillation Fractions of Example 8

| Fraction | Weight (g) | b.p. °C | Normalized Area % 2[a] | 4[b] | bis[c] | tris[d] |
|---|---|---|---|---|---|---|
| 1 | 1.43 | 83-96 | 90.7 | 0.1 | 0.9,7.5 | - |
| 2 | 7.34 | 124-130[e] | 0.5 | - | 99.3 | 0.2 |
| 3 | 6.71 | 130-136[e] | - | - | 93.1 | 6.5 |
| 4 | 2.57 | 127-153[f] | - | - | 30.4 | 69.6 |

[a] 3.5-dimethyl-2-(methythio)phenol
[b] 3.5-dimethyl-4-(methylthio)phenol
[c] 3.5-dimethyl-2.4-bis(methylthio)phenol and 3.5-dimethyl-2.6-bis(methylthio)phenol
[d] 3.5-dimethyl-2.4.6-tris(methylthio)phenol
[e] mainly at 129-130 °C
[f] mainly at 143 °C

NMR shows fraction 2 to be a mixture of about 90 : 10 of 3.5-dimethyl-2.6-bis(methylthio)phenol and 2.4-bis(methylthio)phenol.

Fraction 4 was a yellow liquid that partially crystallized and formed white solid needles having a m. p. of 55-57 °C upon being recrystallized twice from n-hexane. NMR analysis showed the needles to be pure 3,5-dimethyl-2,4,6-tris-(methylthio)phenol.

## Example 9

Preparation of 4-(1,1,3,3-Tetramethylbutyl)-2,6-bis(methylthio)phenol

Triethylaluminum, 0.3 ml (0.002 mole), was added to a mixture of 30.9 g (0.15 mole) of 4-(1,1,3,3-tetramethylbutyl)phenol (Schenectady Chemicals Inc., high purity grade, m. p. of 75-88 °C) and 0.31 g (0.011 mole) of aluminum (8-10 mesh). This mixture was heated at 185-190 °C until all of the aluminum dissolved (about one hour). The mixture was cooled and then 40 ml (0.45 mole) of methyl disulfide was added, and this mixture was heated to reflux.

After six days of reflux the mixture was cooled, poured into 100 ml of 3N HCl and extracted with diethyl ether/pentane. The organic layer was extracted with saturated NaCl and dried over $Na_2SO_4$. Distillation gave a forecut having a b. p. of 100-137 °C/0.1 mm and 27.7 g (62 % yield) of a fraction having a b. p. of 137-141 °C/0.1 mm. NMR showed the fraction to be 4-(1,1,3,3-tetramethylbutyl)-2,6-bis(methylthio)phenol.

## Example 10

Reaction of Methyl Disulfide with 2,6-Dimethylphenol

A mixture of 20 g (0.164 mole) of 2,6-dimethylphenol and 0.31 g (0.011 mole) of 5-10 mesh aluminum was heated at 190 °C for 2 hr during which time the metal dissolved to give a red solution. The solution was cooled to 100 °C and 20 ml (0.22 mole) of methyl disulfide was added. The reaction was refluxed for 2 hr, cooled, an additional 10 ml (0.11 mole) of methyl disulfide was added, and the mixture refluxed overnight. The reaction was cooled, 17 ml (0.19 mole) of methyl disulfide was added, and the reaction refluxed for 24 hr. The reaction was again cooled, 15 ml (0.17 mole) of methyl disulfide was added, and the reaction refluxed for 3 more days.

At the end of the time, the reaction was cooled, stirred with a mixture of 50 ml 1.5N HCl and 100 ml of diethyl ether, and filtered. The greenish-brown solids were rinsed with 50 ml of diethyl ether. The filtrate and wash were combined, the aqueous phase separated, and the aqueous phase extracted once with 50 ml of diethyl ether. The combined organic phases were extracted with 25 ml water, dried ($Na_2SO_4$), filtered and the solvent removed to give 34 g of a reddish-black liquid.

VPC analysis showed 57 area %
2,6-dimethyl-(methylthio)phenol, 29 area %
2,6-dimethyl-bis(methylthio)phenol and 11 area %
2,6-dimethyl-bis(methylthio)phenol.

Distillation through a 7 cm jacketed Vigreux column gave 9.1 g (33 % yield) of a slightly yellow liquid (b. p. 127-130 °C at 3 mm) which crystallized upon standing. Nmr analysis indicated that the distillate was a 74 : 36 mixture of 2,6-dimethyl-4(methylthio)phenol and 2,6-dimethyl-3(methylthio)phenol.

Continued distillation gave 7.4 g (20 % yield) of a yellow liquid (b. p. 159-162 °C/2 mm) which solidified upon standing. Combined VPC and nmr analysis showed that this fraction of the distillation was a 74 : 23 mixture of 2,6-dimethyl-3,4-bis(methylthio)phenol and 2,6-dimethyl-3,5-bis(methylthio)phenol.

Recrystallization of the solid from n-hexane and then $CCl_4$ gave pure 2,6-dimethyl-3,4-bis(methylthio)-phenol as white crystals, m. p. 98-100 °C.

## Example 11

Reaction of 2,4,6-trimethylphenol and Dimethyl Disulfide

To 10.6 g (0.078 mole), of 2,4,6-trimethylphenol was cautiously added, in a nitrogen atmosphere, 1.3 ml (0.005 mole) of triisobutylaluminum. The stirred mixture was heated to 100° and 15 ml (0.166 mole) of methyl disulfide was added. The reaction was then stirred at reflux. Analysis by gas chromatography of an acid-quenched sample showed a composition, after 1 day at reflux, of 86 area % 2,4,6-trimethylphenol and 12 area % 2,4,6-trimethyl-3(methylthio)phenol. Another 7 ml (0.077 mole) of methyl disulfide was added and reflux was continued. An analysis after 24 hours (2 days total) showed 30 area % 2,4,6-trimethylphenol and 66 area % 2,4,6-trimethyl-3(methylthio)phenol. The reaction was refluxed for an additional 5 days (7 days total), cooled, treated with 50 ml 0.1N HCl and extracted 4 × 50 ml $CH_2Cl_2$. The combined extracts were concentrated on a rotary evaporator to give a black oil. Gc analysis showed 7 area % 2,4,6-trimethylphenol, 54 area % 2,4,6-trimethyl-3(methylthio)phenol and 6 area % 2,4,6-trimethyl-3,5-bis(methylthio)phenol. Mass spectroscopy confirmed the identity of the compounds.

## Use

The use of the compounds of this invention as antioxidants is yet another aspect of this invention.

In order to demonstrate the effectiveness of the compounds of this invention as antioxidants, a variety of these novel compounds were blended with lubricating oil and the resultant blends subjected to a bench test which measures the extent of oxidative decomposition and bearing corrosion experienced at elevated temperatures in the presence of a typical oxidation catalyst.

In particular, the antioxidant properties were tested by blowing air at the rate of 0.2 liters/min. through a base oil, Texaco Ursa P-20, 100 g, at 160 °C for 18 hours to which had been added enough iron naphthenate (a soluble oxidation catalyst) to provide 0.005 % by weight iron. A half shell Cu/Pb engine bearing was also present in the oil during the test. The results of these tests are given in Table V. Additives (2) to (9) are compounds of this invention. Additive (10) is a commercially available antioxidant from Ethyl Corporation. The results show the viscosity increase in percent and the bearing weight loss (B.W.L.) in mg.

Table V

Bench Oxidation — Corrosion Test Results

| Additive | % by Wt. | Viscosity Increase % | B.W.L. (mg.) |
|---|---|---|---|
| ( 1) None | - | 829 | 91 |
| ( 2) A mixture of 4-nonyl-2,6-bis-(methylthio)phenol and 6-nonyl-2,4-bis-(methylthio)phenol | 1.0 | 69 | 97 |
| ( 3) 4-*tert*-butyl-2,6-bis-(methylthio) phenol | 1.0 | 70 | 107 |
| ( 4) 4-phenyl-2,6-bis(methylthio)phenol | 1.0 | 56 | 14 |
| ( 5) 4-methyl-2,6-bis(methylthio)phenol | 1.0 | 6 | 20 |
| ( 6) 2,4,6-tris(methylthio)-phenol | 1.0 | -2 | 6 |
| ( 7) 2,6-bis(methylthio)phenol | 1.0 | 17 | 9 |
| ( 8) 4-phenyl-2,6-bis(methylthio)phenol | 0.5 | 105 | 56 |
| ( 9) 4-methyl-2,6-bis(methylthio)phenol | 0.5 | 12 | 15 |
| (10) 4,4'-methylenebis(2,6-di-*tert*-butylphenol)* | 1.0 | 68 | 111 |

*An antioxidant commercially available from Ethyl Corporation under the product designation ETHYL 702 Antioxidant.

Results of a sludge rating for (1)-(5) of Table V were D and the rating for (10) was C. A Sludge rating is a subjective determination of the amount of residue left on the sides of the test tubes after the Bench Oxidation-Corrosion Test is run. The ratings are as follows : A = Clean, B = Very Light, C = Light, D = Heavy and E = Very Heavy.

The results shown in Table V demonstrate the effectiveness of a number of compounds of this invention as antioxidants.

Another series ot test was carried out following the above procedure. The results of these tests are given in Table VI.

Table VI

Bench Oxidation — Corrosion Test Results

| | Additive | % by Wt. | Viscosity Increase % | B.W.L. (mg.) |
|---|---|---|---|---|
| (1) | None | 1.0 | 619 | 132 |
| (2) | 4-phenyl-2,6-bis(methylthio)phenol | 1.0 | 9 | 10 |
| (3) | 4-*tert*-butyl-2,6-bis(methylthio) phenol | 1.0 | 53 | 64 |
| (4) | A mixture of 3,5-dimethyl-2,6-bis(methylthio)phenol and 3,5-dimethyl-2,4-bis-(methylthio)phenol* | 1.0 | 75 | 82 |
| (5) | A mixture of 2,2',6,6'-tetra-(methylthio)-4,4'-isopropylidenediphenol** | 1.0 | 16 | -3 |
| (6) | A mixture of 2,6-dimethyl-3,4-bis(methylthio)phenol and 2,6-dimethyl-3,5-bis-(methylthio)phenol*** | 1.0 | 45 | 83 |

*About 90 % by wt. of the 2,6-bis isomer and about 10 % by wt. of the 2,4-bis isomer.
**About 60 % by wt. of the 2,2',6,6'-tetra isomer and about 35 % by wt. of the 2,2',6-tris isomer.
***About 74 % by wt. of the 3,4-bis isomer and about 23 % by wt. of the 3,5-bis isomer.

In the tests of Table VI the oil without any additive had a Sludge rating of E+ whereas additives (2)-(6) had sludge ratings of E.

The results in Table VI once again show that the compounds of this invention are effective as antioxidants.

From the results of these tests it can be seen that the compounds of this invention will exhibit antioxidant properties in a variety of substrates when used with the substrate in small concentrations, usually concentrations below 2 % by weight of the substrate, preferably within the range of from 0.1 % by weight to 1 % by weight and more preferably within the range of from 0.25 % by weight to 0.75 % by weight, typically 0.5 % by weight. As is well understood in the art, the particular concentration of an antioxidant employed will be governed to some extent by the nature of the substrate and the type and severity of the storage and service conditions to which the substrate will be exposed. Thus when using the compounds of this invention as antioxidants, the amounts may be varied to suit the needs of the occasion.

The substrates that may be protected by means of the antioxidant compounds of this invention include a broad range of organic materials of the type normally subject to oxidative deterioration in the presence of oxygen during storage or use over an extended period. In other words, the organic compositions protected by the present antioxidants are the type in which the art recognizes the need for antioxidant protection and to which an antioxidant of some type is customarily added to obtain an extended service life. The oxidative degradation protected against is the slow gradual deterioration of the organic composition rather than, for example, combustion. In other words, the present additives are not flame retarding agents nor flame suppressing additives and the degradation protected against is not combustion but, rather, the gradual deterioration of the organic composition due to the effects of oxygen over an extended period of time.

Examples of organic materials in which the additives, i. e. the products of this invention may be useful include homopolymers and copolymers of olefinically unsaturated monomers, for example, polyolefins such as polyethylene, polypropylene, polybutadiene, and the like. Also, poly-halohydrocarbons such as polyvinyl chloride, polychloroprene, polyvinylidene chloride, polyfluoro olefins, and the like, are afforded stabilization. The additives may provide both antioxidant and antiozonate protection in natural and synthetic rubbers such as copolymers of olefinically unsaturated monomers including styrene-butadiene rubber (SBR rubber), ethylene-propylene copolymers, ethylene-propylene-diene terpolymers such as the terpolymer of ethylene, propylene and cyclopentadiene or cyclooctadiene. Polybutadiene rubbers such as cis-polybutadiene rubber may be protected. Poly-2-chloro-1,3-butadiene (neoprene) and poly-2-methyl-1,3-butadiene (isoprene rubber) may be stabilized by the present additives. Likewise, acrylonitrile-butadiene-styrene resins and polystyrenes (both crystal grades and rubber modified grades) are

effectively stabilized. Ethylene-vinyl acetate copolymers may be protected, as are butene-methylacrylate copolymers. Nitrogen-containing polymers such as polyurethanes, nitrile rubber, and lauryl acrylate-vinyl-pyrrolidone copolymers may be effectively stabilized. Adhesive compositions such as solutions of polychloroprene (neoprene) in toluene may be protected.

Petroleum oils and waxes such as solvent-refined mid-continent lubricating oil, microcrystalline wax, and Gulf-coast lubricating oils are effectively stabilized. The additives may be useful in foamed plastics such as expanded polystyrene, polyurethane foams, and the various foamed rubbers, alkyd resins such as short oil terephthalic acid-glycerol-linseed oil resins, and resins including epoxide-modified alkyl resins. Epoxy resins themselves such as isopropylidenebisphenol-epichlorohydrin epoxy resins may be stabilized against degradation.

Hydrocarbons such as gasoline, kerosene, diesel fuel, fuel oil, furnace oil, and jet fuel may be effectively protected. Likewise, synthetic hydrocarbon lubricants, for example, α-decene trimer, polybutene lubricants, di- and tri-$C_{12-30}$ alkylated benzene and naphthalene synthetic lubricants are likewise protected.

Organometallics such as tetraethyllead, tetramethyllead, tetravinyllead, ferrocene, methyl ferrocene, cyclopentadienyl magnesium tricarbonyl, methyl cyclopentadienyl magnesium tricarbonyl, cyclopentadienyl nickel nitrosyl, and the like, may be effectively protected against oxidative degradation. Silicone oils and greases may be also protected.

Synthetic ester lubricants such as those used in turbines and turbojet engines may be given a high degree of stabilization. Typical synthetic ester lubricants include di-2-ethylhexyl sebacate, trimethylolpropane tripelargonate, $C_{5-9}$ aliphatic monocarboxylic esters of pentaerythritol, complex esters formed by condensing under esterifying conditions mixtures of polyols, polycarboxylic acids, and aliphatic monocarboxylic acids and/or monohydric alkanols. An example of these complex esters is the condensation product formed from adipic acid, ethylene glycol and a mixture of $C_{5-9}$ aliphatic monocarboxylic acids. Plasticizers such as dioctyl phthalate may be effectively protected. Heavy petroleum fractions such as tar and asphalt may also be protected should the need arise.

Polyamides such as adipic acid-1,6-diaminohexane condensates and poly-6-aminohexanoic acid (nylon) may be effectively stabilized. Polyalkylene oxides such as copolymers of phenol with ethylene oxide or propylene oxide may be stabilized. Polyphenylene ethers such as poly-2,6-dimethylphenylene ether formed by polymerization of 2,6-dimethylphenol using a copper-pyridine or like catalyst may be stabilized. Polycarbonate plastics and polyformaldehyde resins may be also protected.

Linear polyesters such as phthalic anhydride-glycol condensates may be given a high degree of protection. Other polyesters such as trimellitic acid-glycerol condensates may be also protected. Polyacrylates such as polymethylacrylate and polymethylmethacrylate may be effectively stabilized. Polyacrylonitriles and copolymers of acrylonitriles with other olefinically unsaturated monomers such as methylmethacrylates may be also effectively stabilized.

The additives may be used to protect any of the many organic substrates to which an antioxidant is normally added. It may be used where economics permit to protect such substrates as asphalt, fluorocarbons such as Teflon, polyvinyl acetate, polyvinylidene chloride, coumarone-indene resins, polyvinyl ethers, polyvinylidene bromide, polyvinylbromide, acrylonitrile, vinyl bromide copolymer, vinyl butyral resins, silicones such as dimethylsilicone lubricants, phosphate lubricants such as tricresylphosphate, and the like.

Methods of incorporating the additive into the substrate are well known. For example, if the substrate is liquid the additive can be merely mixed into the substrate. Frequently the organic substrate is in solution and the additive is added to the solution and the solvent removed. Solid organic substrates can be merely sprayed with a solution of the additive in a volatile solvent. For example, stabilized grain products result from spraying the grain with a toluene solution of the additive. In the case of rubbery polymers the additive can be added following the polymerization stage by mixing it with the final emulsion or solution polymerization mixture and then coagulating or removing solvent to recover the stabilized polymer. It can also be added at the compounding stage by merely mixing the additive with the rubbery polymer in commercial mixing equipment such as a Banbury blender. In this manner, rubbery polymers such as styrene-butadiene rubber, cis-polybutadiene or isoprene polymers are blended with the antioxidant together with the other ingredients normally added such as carbon black, oil, sulfur, zinc oxide, stearic acid, vulcanization accelerators, and the like. Following mastication, the resultant mixture is fabricated and molded into a finished form and vulcanized.

Other utilities for the compounds of this invention include their use as raw materials in the synthesis of polymers and as intermediates in the synthesis of pharmaceuticals, pesticides, herbicides, and the like.

**Claims** (for the Contracting States : BE, CH, DE, FR, GB, IT, LI, LU, NL, SE)

1. A compound represented by one of the following formulas :

(See formula I p. 19)

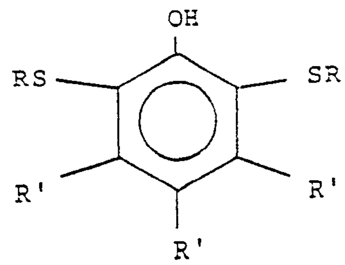

(I)

wherein in Formula I at least two of the R′ groups are the same or different hydrocarbyl groups devoid of olefinic and acetylenic unsaturation, and the remaining R′ group is hydrogen or a hydrocarbyl group devoid of olefinic and acetylenic unsaturation, and wherein R is a hydrocarbyl group having up to 10 carbon atoms ;

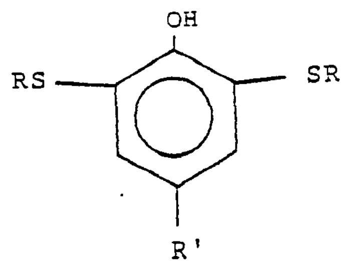

(II)

wherein the R′ group in Formula II is hydrogen or a hydrocarbyl group devoid of olefinic and acetylenic unsaturation, and wherein R is a hydrocarbyl group having up to 10 carbon atoms ;

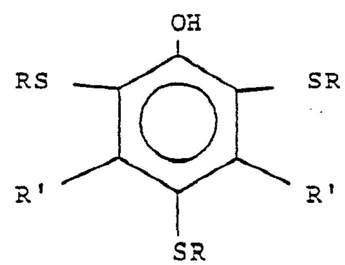

(III)

wherein the R′ groups in Formula III may be the same or different, and are hydrogen atoms or hydrocarbyl groups devoid of olefinic and acetylenic unsaturation, and wherein R is a hydrocarbyl group having up to 10 carbon atoms ;

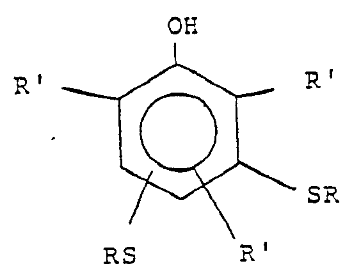

(IV)

wherein in Formula IV, the R′ groups in the 2- and 6-positions are the same or different hydrocarbyl groups devoid of olefinic and acetylenic unsaturation, while the other R′ group is a hydrogen atom or a hydrocarbyl group devoid of olefinic and acetylenic unsaturation, which hydrocarbyl group can be the same as or different from the hydrocarbyl groups in the 2- and 6-positions and wherein R is a hydrocarbyl group having up to 10 carbon atoms ;

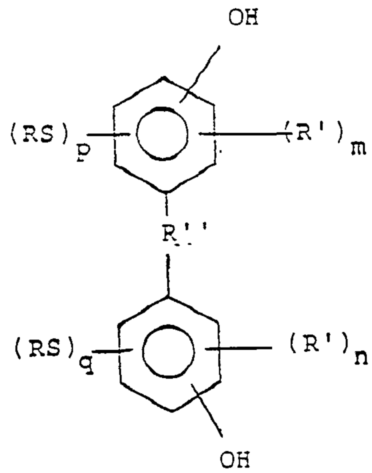

(VI)

wherein the R′ groups in Formula VI may be the same or different, and are hydrogen atoms or hydrocarbyl groups devoid of olefinic and acetylenic unsaturation ; wherein R″ is an alkylidene or n alkylene group of from 1 to 10 carbon atoms ; wherein m and n are integers from 0 to 4 (both inclusive), p and q.are integers from 0 to 4 (both inclusive) and either or both of p and q is an integer of at least 2, the total of m and p and the total of n and q each being 4 ; and wherein R is a hydrocarbyl group containing up to 10 carbon atoms ;

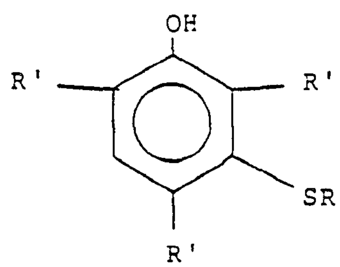

(VIII)

or

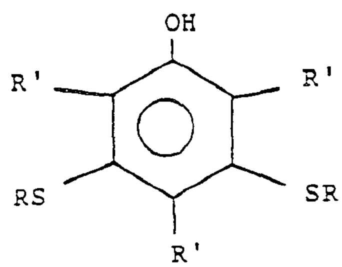

(IX)

or

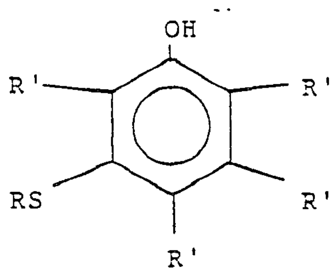

(X)

wherein in Formulas VIII, IX and the R′ groups are the same or different hydrocarbyl groups and R is a hydrocarbyl group having up to 10 carbon atoms.

2. A compound as claimed in Claim 1 wherein R′ groups are alkyl groups of 1 to 50 carbon atoms.

3. A compound as claimed in Claim 2 wherein R is an alkyl group containing up to about 4 carbon atoms.

4. 2,4,6-trimethyl-3-(methylthio)phenol.

5. 2,4,6-trimethyl-3,5-bis(methylthio)phenol.

6. A compound as claimed in Formula VI of Claim 1 of Formula VI wherein the hydroxyl groups of said compound are in the 4 and 4′ positions relative to R″.

7. A compound as claimed in Claim 6, namely 2,2',6,6'-tetra(methylthio)-4,4'-isopropylidene-diphenol.

8. The compound as claimed in Claim 7 in admixture with 2,2',6-tris-(methylthio)-4,4'-iso-propylidenediphenol.

9. A composition comprising an organic material normally susceptible to oxidative degradation containing a (hydrocarbylthio)phenol according to claim 1 in an amount sufficient to retard said degradation.

10. A composition as claimed in claim 9 wherein said organic material is a mineral oil.

11. A process for preparing a compound of claim 1, characterized in that it comprises reacting an excess of hydrocarbyl disulfide with one molar proportion of a phenol which is free of substitution on the positions to be substituted by hydrocarbylthio groups in the presence of less than one molar proportion of an aluminum phenoxide catalyst at a temperature of 0-300 °C.

**Claims** (for the Contracting State AT)

1. A composition comprising an organic material normally susceptible to oxidative degradation containing a (hydrocarbylthio)phenol in an amount sufficient to retard said degradation, said phenol having one of the following formulae

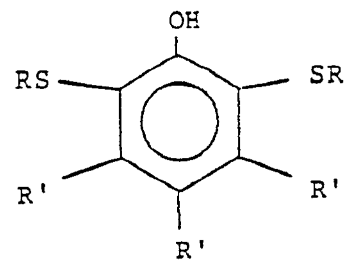

(I)

wherein in Formula I at least two of the R' groups are the same or different hydrocarbyl groups devoid of olefinic and acetylenic unsaturation, and the remaining R' group is hydrogen or a hydrocarbyl group devoid of olefinic and acetylenic unsaturation, and wherein R is a hydrocarbyl group having up to 10 carbon atoms ;

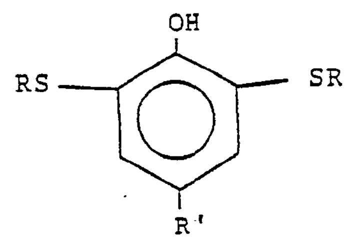

(II)

wherein the R' group in Formula II is hydrogen or a hydrocarbyl group devoid of olefinic and acetylenic unsaturation, and wherein R is a hydrocarbyl group having up to 10 carbon atoms ;

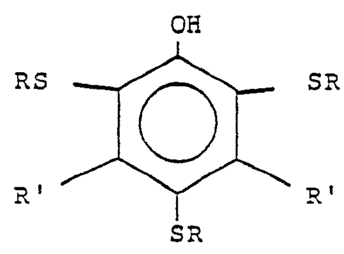

(III)

wherein the R' groups in Formula III may be the same or different, and are hydrogen atoms or hydrocarbyl groups devoid of olefinic and acetylenic unsaturation, and wherein R is a hydrocarbyl group having up to 10 carbon atoms ;

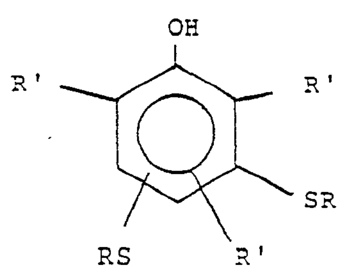

(IV)

wherein in Formula IV, the R′ groups in the 2- and 6-positions are the same or different hydrocarbyl groups devoid of olefinic and acetylenic unsaturation, while the other R′ group is a hydrogen atom or a hydrocarbyl group devoid of olefinic and acetylenic unsaturation, which hydrocarbyl group can be the same as or different from the hydrocarbyl groups in the 2- and 6-positions and wherein R is a hydrocarbyl group having up to 10 carbon atoms ;

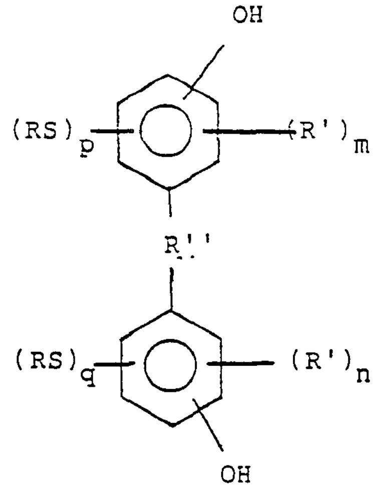

(VI)

wherein the R′ groups in Formula VI may be the same or different, and are hydrogen atoms or hydrocarbyl groups devoid of olefinic and acetylenic unsaturation ; wherein R″ is an alkylidene or an alkylene group of from 1 to 10 carbon atoms ; wherein m and n are integers from 0 to 4 (both inclusive), p and q are integers from 0 to 4 (both inclusive) and either or both of p and q is an integer of at least 2, the total of m and p and the total of n and q each being 4 ; and wherein R is a hydrocarbyl group containing up to 10 carbon atoms ;

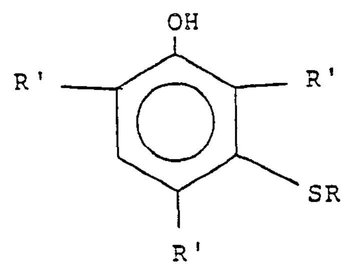

(VIII)

or

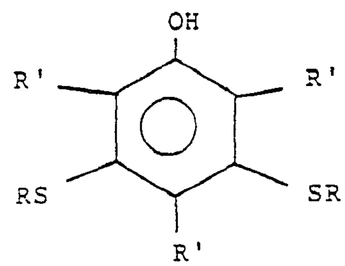

(IX)

or

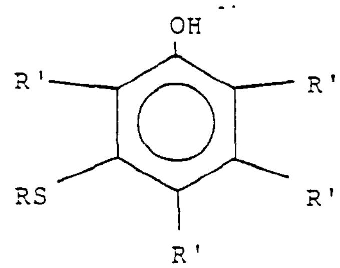

(X)

wherein in Formulas VIII, IX and X the R' groups are the same or different hydrocarbyl groups and R is a hydrocarbyl group having up to 10 carbon atoms.

2. A composition as claimed in Claim 1 wherein said organic material is a mineral oil.

3. A process for preparing a hydrocarbylthio phenol as defined in claim 1, characterized in that it comprises reacting an excess of hydrocarbyl disulfide with one molar proportion of a phenol which is free of substitution on the positions to be substituted by hydrocarbylthio groups in the presence of less than one molar proportion of an aluminum phenoxide catalyst at a temperature of 0-300 °C.

**Patentansprüche** (für die Vertragsstaaten : BE, CH, DE, FR, GB, IT, LI, LU, NL, SE)

1. Verbindung der folgenden Formeln

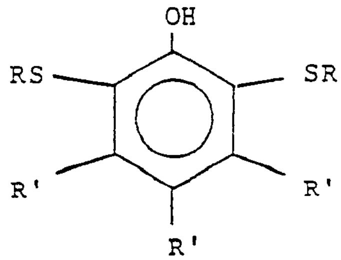

(I)

worin in der Formel (I) mindestens 2 der Gruppen R' gleiche oder verschiedene Hydrocarbylgruppen ohne olefinische und acetylenische Unsättigung bedeuten und die verbleibende Gruppe R' Wasserstoff oder eine Hydrocarbylgruppe ohne olefinische und acetylenische Unsättigung ist und R eine Hydrocarbylgruppe mit bis zu 10 Kohlenstoffatomen bedeutet ;

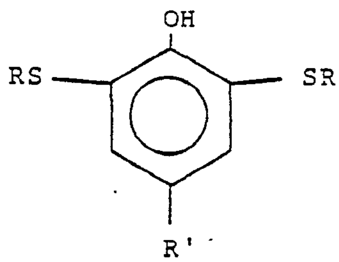

(II)

worin die Gruppe R' in der Formel (II) Wasserstoff oder eine Hydrocarbylgruppe ohne olefinische und acetylenische Unsättigung bedeutet und R eine Hydrocarbylgruppe mit bis zu 10 Kohlenstoffatomen bedeutet ;

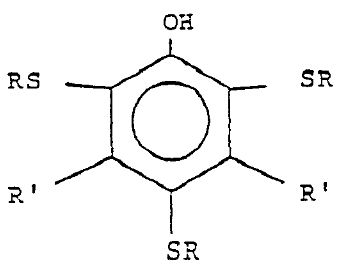

(III)

worin die Gruppen R' in der Formel (III), die gleich oder verschieden sind, Wasserstoffatome oder Hydrocarbylgruppen ohne olefinische und acetylenische Unsättigung bedeuten und R eine Hydrocarbylgruppe mit bis zu 10 Kohlenstoffatomen bedeutet ;

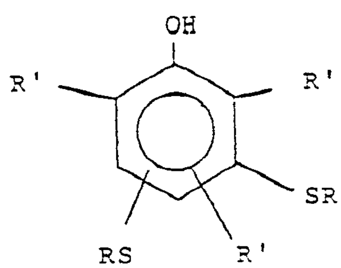

(IV)

worin in Formel (IV) die Gruppen R' in der 2- und 6-Stellung gleiche oder verschiedene Hydrocarbylgruppen ohne olefinische und acetylenische Unsättigung bedeuten, während die übrige Gruppe R' ein Wasserstoffatom oder eine Hydrocarbylgruppe ohne olefinische und acetylenische Unsättigung bedeutet, wobei diese Hydrocarbylgruppe gleich oder verschieden von den Hydrocarbylgruppen in den 2- und 6-Stellungen sein kann und R eine Hydrocarbylgruppe mit bis zu 10 Kohlenstoffatomen bedeutet ;

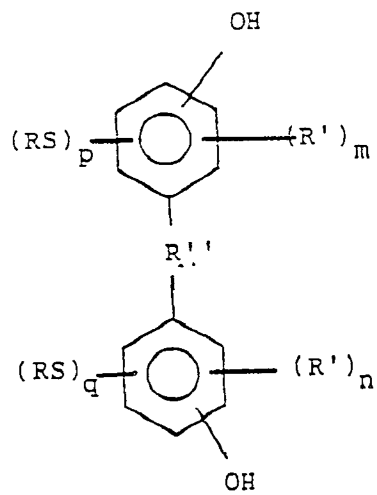

(VI)

worin die Gruppen R' in Formel (VI) gleich oder verschieden sind und Wasserstoffatome oder Hydrocarbylgruppen ohne olefinische und acetylenische Unsättigung bedeuten, R'' eine Alkyliden- oder eine Alkylengruppe mit 1 bis 10 Kohlenstoffatomen bedeutet ; m und n eine ganze Zahl von 0 bis 4 (einschließlich), p und q eine ganze Zahl von 0 bis 4 (einschließlich) ist und entweder eine Zahl oder beide Zahlen von p und q ist mindestens 2, wobei die Gesamtzahl von m und p und die Gesamtzahl von n und q jeweils 4 ist und R eine Hydrocarbylgruppe mit bis zu 10 Kohlenstoffatomen bedeutet ;

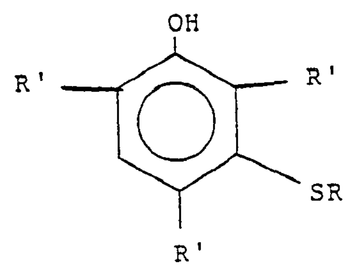

(VIII)

oder

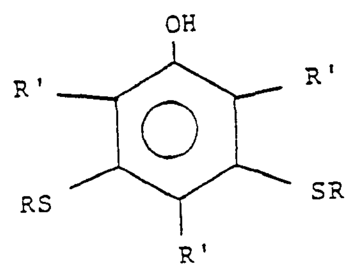

(IX)

oder

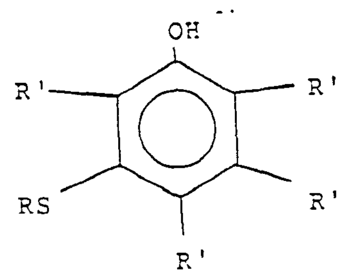

(X)

worin in den Formeln (VIII), (IX) und (X) die Gruppen R' gleiche oder verschiedene Hydrocarbylgruppen sind und R eine Hydrocarbylgruppe mit bis zu 10 Kohlenstoffatomen bedeutet.

2. Verbindung nach Anspruch 1, worin die Gruppen R' Alkylgruppen mit 1 bis 50 Kohlenstoffatomen sind.

3. Verbindung nach Anspruch 2, worin R eine Alkylgruppe mit bis zu etwa 4 Kohlenstoffatomen ist.

4. 2,4,6-Trimethyl-3-(methylthio)phenol.

5. 2,4,6-Trimethyl-3,5-bis(methylthio)phenol.

6. Verbindung, gemäß Formel (VI) in Anspruch 1 der Formel (VI), worin die Hydroxylgruppen der Verbindung in der 4- und 4'-Stellung relativ zur R" stehen.

7. Verbindung nach Anspruch 6, nämlich 2,2',6,6'-Tetra(methylthio)-4,4'-isopropyliden-diphenol.

8. Verbindung nach Anspruch 7, als Beimischung zu 2,2',6-Tris-(methylthio)-4,4'-iso-propylidendiphenol.

9. Zubereitung, enthaltend ein normalerweise für den oxidativen Abbau anfälliges organisches Material, enthaltend ein (Hydrocarbylthio)phenol nach Anspruch 1, in einer Menge, die ausreicht, den Abbau zu hemmen.

10. Zubereitung nach Anspruch 9, in der das organische Material ein Mineralöl ist.

11. Verfahren zur Herstellung einer Verbindung nach Anspruch 1, dadurch gekennzeichnet, daß man einen Überschuß von Hydrocarbyldisulfid mit einem 1-molaren Anteil eines Phenols, das frei von einer Substitution in den Stellungen, die durch Hydrocarbylthiogruppen substituiert werden, ist, in Gegenwart von weniger als einem molaren Anteil eines Aluminiumphenoxidkatalysators bei einer Temperatur von 0 bis 300 °C umsetzt.

**Patentansprüche** (für den Vertragsstaat AT)

1. Zubereitung, enthaltend ein für einen oxidativen Abbau normalerweise empfängliches organisches Material, enthaltend ein (Hydrocarbylthio)phenol, in einer Menge, die ausreicht, den Abbau zu hemmen, wobei das Phenol eine der folgenden Formeln aufweist

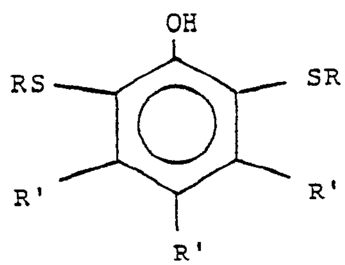

(I)

worin in der Formel (I) zumindest 2 der Gruppen R' gleiche oder verschiedene Hydrocarbylgruppen ohne olefinische und acetylenische Unsättigung sind und die verbleibende Gruppe R' Wasserstoff oder eine Hydrocarbylgruppe ohne olefinische und acetylenische Unsättigung bedeutet und R eine Hydrocarbylgruppe mit bis zu 10 Kohlenstoffatomen bedeutet ;

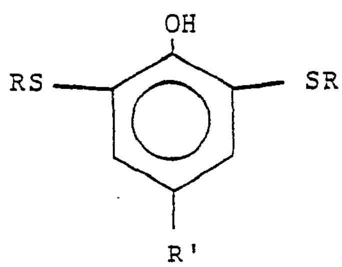

(II)

worin in Formel (II) die Gruppe R' Wasserstoff oder eine Hydrocarbylgruppe ohne olefinische und acetylenische Unsättigung und R eine Hydrocarbylgruppe mit bis zu 10 Kohlenstoffatomen bedeutet ;

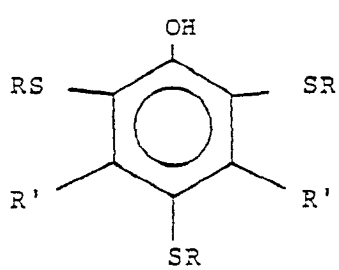

(III)

worin die Gruppen R' in der Formel (III) gleich oder verschieden sind und Wasserstoffatome oder Hydrocarbylgruppen ohne olefinische und acetylenische Unsättigung bedeuten und R eine Hydrocarbylgruppe mit bis zu 10 Kohlenstoffatomen bedeutet ;

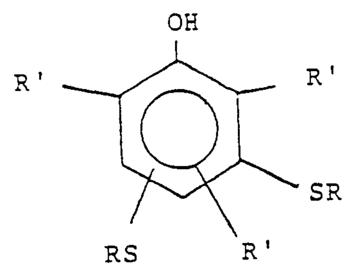

(IV)

worin in Formel (IV) die Gruppen R' in den 2- und 6-Stellungen gleiche oder verschiedene Hydrocarbylgruppen ohne olefinische und acetylenische Unsättigung bedeuten, während die übrige Gruppe R' ein Wasserstoffatom oder eine Hydrocarbylgruppe ohne olefinische und acetylenische Unsättigung bedeutet, wobei diese Hydrocarbylgruppe gleich oder verschieden von den Hydrocarbylgruppen in den 2- und 6-Stellungen sein kann und R eine Hydrocarbylgruppe mit bis zu 10 Kohlenstoffatomen bedeutet ;

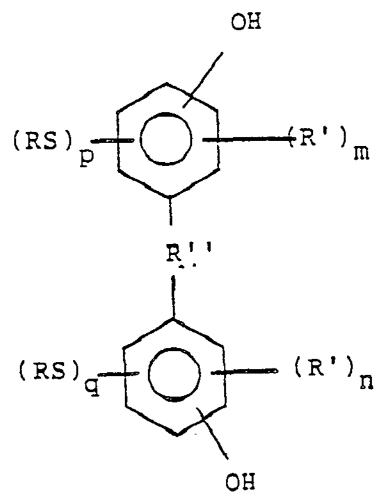

(VI)

worin die Gruppen R' in der Formel (VI) gleich oder verschieden sind und Wasserstoffatome oder Hydrocarbylgruppen ohne olefinische und acetylenische Unsättigung bedeuten, R'' eine Alkyliden- oder eine Alkylengruppe mit 1 bis 10 Kohlenstoffatomen bedeutet und m und n eine ganze Zahl von 0 bis 4 (einschließlich), p und q eine ganze Zahl von 0 bis 4 (einschließlich) und entweder eine Zahl oder beide Zahlen aus p und q eine ganze Zahl von Zumindest 2 ist, die Gesamtzahl aus m und p und die Gesamtzahl aus n und q jeweils 4 ist und R eine Hydrocarbylgruppe mit bis zu 10 Kohlenstoffatomen bedeutet ;

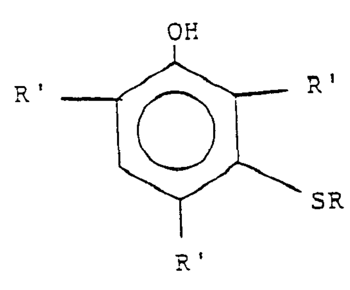

(VIII)

oder

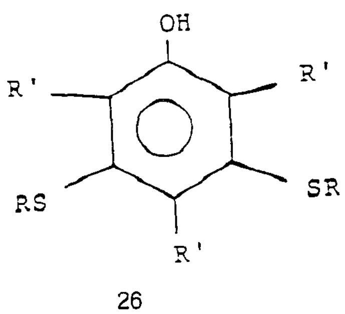

(IX)

oder

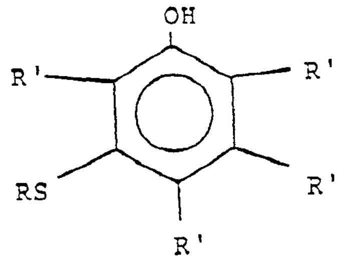

(X)

worin in den Formeln (VIII), (IX) und (X) die Gruppen R' gleiche oder verschiedene Hydrocarbylgruppen und R eine Hydrocarbylgruppe mit bis zu 10 Kohlenstoffatomen bedeutet.

2. Zubereitung nach Anspruch 1, in der das organische Material ein Mineralöl ist.

3. Verfahren zur Herstellung von Hydrocarbylthiophenol, wie in Anspruch 1 definiert, dadurch gekennzeichnet, daß man einen Überschuß von Hydrocarbyldisulfid mit einem 1-molaren Anteil eines Phenols, das frei von Substitution an den Stellungen, die durch die Hydrocarbylthiogruppen substituiert werden, ist, in Gegenwart von weniger als einer molaren Menge eines Aluminiumphenoxidkatalysators bei einer Temperatur von 0 bis 300 °C umsetzt.

**Revendications** (pour les Etats contractants : BE, CH, DE, FR, GB, IT, LI, LU, NL, SE)

1. Composé représenté par l'une des formules suivantes :

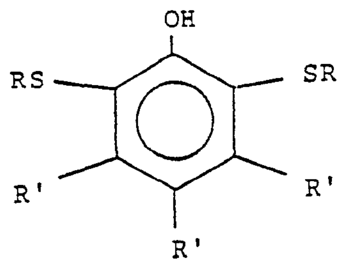

(I)

formule I dans laquelle au moins deux des groupes R' sont des groupes hydrocarbyle identiques ou différents, dépourvus d'insaturation oléfinique et acétylénique et le groupe R' restant est l'hydrogène ou un groupe hydrocarbyle dépourvu d'insaturation oléfinique et acétylénique, et R est un groupe hydrocarbyle ayant jusqu'à 10 atomes de carbone ;

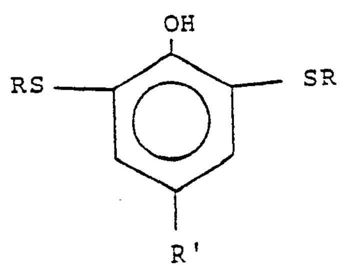

(II)

formule II dans laquelle le groupe R' est un atome d'hydrogène ou un groupe hydrocarbyle dépourvu d'insaturation oléfinique et acétylénique, et R est un groupe hydrocarbyle ayant jusqu'à 10 atomes de carbone ;

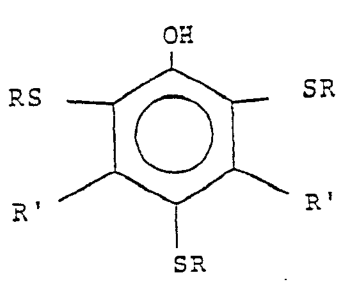

(III)

formule III dans laquelle les groupes R' peuvent être identiques ou différents et sont des atomes d'hydrogène ou des groupes hydrocarbyle dépourvus d'insaturation oléfinique et acétylénique, et R est un groupe hydrocarbyle ayant jusqu'à 10 atomes de carbone ;

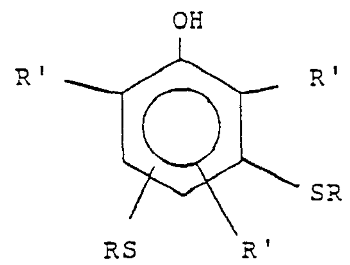

(IV)

formule IV dans laquelle les groupes R' en positions 2 et 6 sont des groupes hydrocarbyle identiques ou différents dépourvus d'insaturation oléfinique et acétylénique, tandis que l'autre groupe R' est un atome d'hydrogène ou un groupe hydrocarbyle dépourvu d'insaturation oléfinique et acétylénique, ce groupe hydrocarbyle pouvant être identique aux groupes hydrocarbyle en positions 2 et 6 ou pouvant en être différent, et R est un groupe hydrocarbyle ayant jusqu'à 10 atomes de carbone ;

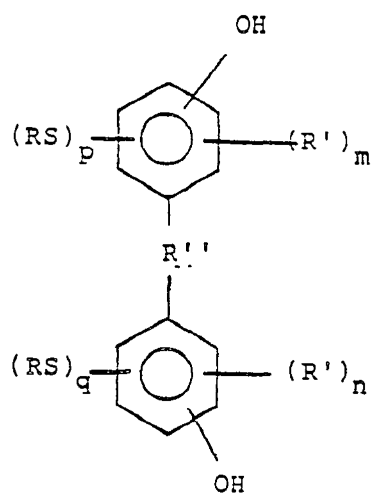

(VI)

formule VI dans laquelle les groupes R' peuvent être identiques ou différents et sont des atomes d'hydrogène ou des groupes hydrocarbyle dépourvus d'insaturation oléfinique et acétylénique ; R'' est un groupe alkylidène ou alkylène ayant 1 à 10 atomes de carbone ; m et n sont des nombres entiers de 0 à 4 (inclus), p et q sont des nombres entiers de 0 à 4 (inclus) et l'un ou l'autre de p et q ou les deux représentent un nombre entier au moins égal à 2, le total de m et p et le total de n et q étant dans chaque cas égal à 4 ; et R est un groupe hydrocarbyle contenant jusqu'à 10 atomes de carbone ;

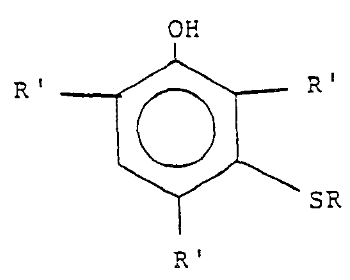

(VIII)

ou

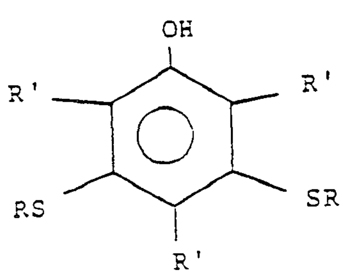

(IX)

ou

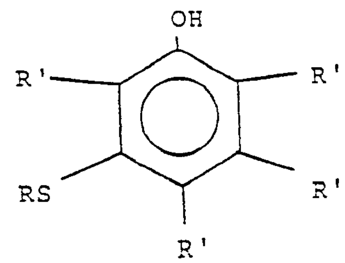

(X)

formules VIII, IX et X dans lesquelles les groupes R' sont des groupes hydrocarbyle identiques ou différents et R est un groupe hydrocarbyle ayant jusqu'à 10 atomes de carbone.

2. Composé suivant la revendication 1, dans lequel les groupes R' sont des groupes alkyle de 1 à 50 atomes de carbone.

3. Composé suivant la revendication 2, dans lequel R est un groupe alkyle contenant jusqu'à environ 4 atomes de carbone.

4. Le 2,4,6-triméthyl-3-(méthylthio)phénol.

5. Le 2,4,6-triméthyl-3,5-bis-(méthylthio)-phénol.

6. Composé de formule VI suivant la revendication 1, formule VI dans laquelle les groupes hydroxyle dudit composé sont en positions 4 et 4' par rapport à R".

7. Composé suivant la revendication 6, à savoir le 2,2',6,6'-tétra(méthylthio)-4,4'-isopropylidène-diphénol.

8. Composé suivant la revendication 7 en mélange avec le 2,2',6-tris-(méthylthio)-4,4'-isopropylidène-diphénol.

9. Composition comprenant une matière organique normalement susceptible de dégradation par oxydation, contenant un (hydrocarbylthio)phénol suivant la revendication 1 en une quantité suffisante pour retarder ladite dégradation.

10. Composition suivant la revendication 9, dans laquelle ladite matière organique est une huile minérale.

11. Procédé de préparation d'un composé suivant la revendication 1, caractérisé en ce qu'il consiste à faire réagir un excès de disulfure d'hydrocarbyle avec une proportion molaire d'un phénol qui est dépourvu de substitution sur les positions devant être substituées par des groupes hydrocarbylthio en présence de moins d'une proportion molaire d'un phénate d'aluminium catalyseur, à une température de 0 à 300 °C.

**Revendications** (pour l'Etat contractant AT)

1. Composition comprenant une matière organique normalement susceptible d'une dégradation par oxydation, contenant un (hydrocarbylthio)phénol en une quantité suffisante pour retarder ladite dégradation, ledit phénol répondant à l'une des formules suivantes :

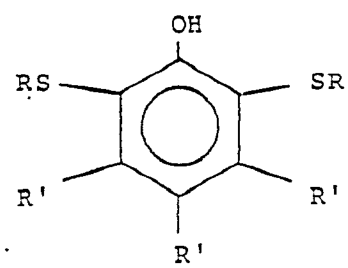

(I)

formule I dans laquelle au moins deux des groupes R' sont des groupes hydrocarbyle identiques ou différents, dépourvus d'insaturation oléfinique et acétylénique et le groupe R' restant est l'hydrogène ou un groupe hydrocarbyle dépourvu d'insaturation oléfinique et acétylénique, et R est un groupe hydrocarbyle ayant jusqu'à 10 atomes de carbone ;

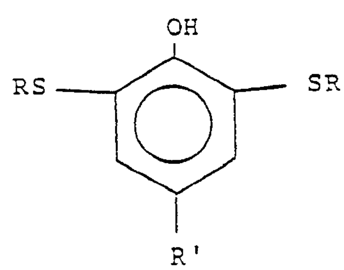

(II)

formule II dans laquelle le groupe R' est un atome d'hydrogène ou un groupe hydrocarbyle dépourvu d'insaturation oléfinique et acétylénique, et R est un groupe hydrocarbyle ayant jusqu'à 10 atomes de carbone ;

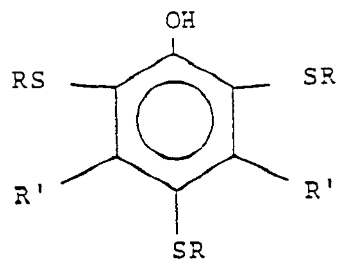

(III)

formule III dans laquelle les groupes R' peuvent être identiques ou différents et sont des atomes d'hydrogène ou des groupes hydrocarbyle dépourvus d'insaturation oléfinique et acétylénique, et R est un groupe hydrocarbyle ayant jusqu'à 10 atomes de carbone ;

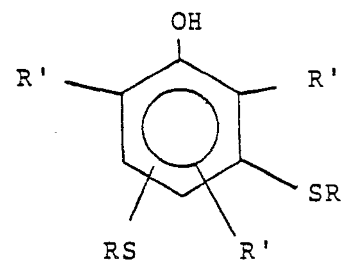

(IV)

formule IV dans laquelle les groupes R' en positions 2 et 6 sont des groupes hydrocarbyle identiques ou différents dépourvus d'insaturation oléfinique et acétylénique, tandis que l'autre groupe R' est un atome d'hydrogène ou un groupe hydrocarbyle dépourvu d'insaturation oléfinique et acétylénique, ce groupe hydrocarbyle pouvant être identique aux groupes hydrocarbyle en postions 2 et 6 ou pouvant en être différent, et R est un groupe hydrocarbyle ayant jusqu'à 10 atomes de carbone ;

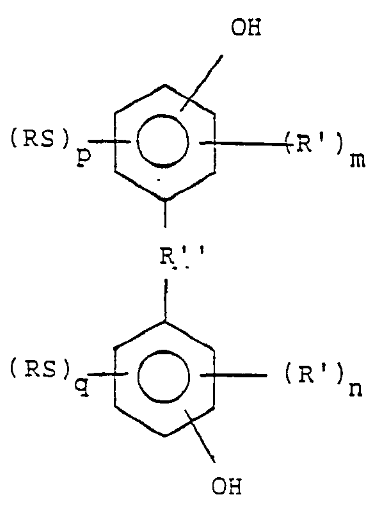

(VI)

formule VI dans laquelle les groupes R' peuvent être identiques ou différents et sont des atomes d'hydrogène ou des groupes hydrocarbyle dépourvus d'insaturation oléfinique et acétylénique ; R'' est un groupe alkylidène ou alkylène ayant 1 à 10 atomes de carbone ; m et n sont des nombres entiers de 0 à 4 (inclus), p et q sont des nombres entiers de 0 à 4 (inclus) et l'un ou l'autre de p et q ou les deux représentent un nombre entier au moins égal à 2, le total de m et p et le total de n et q étant dans chaque cas égal à 4 ; et R est un groupe hydrocarbyle contenant jusqu'à 10 atomes de carbone ;

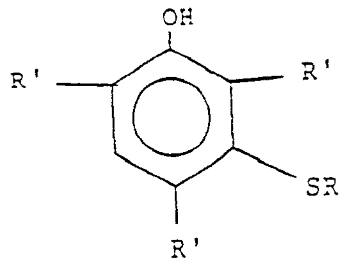

(VIII)

ou

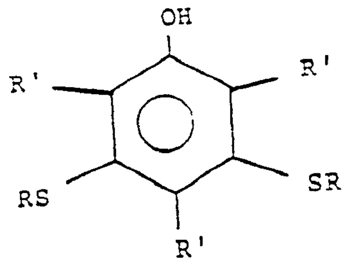

(IX)

ou

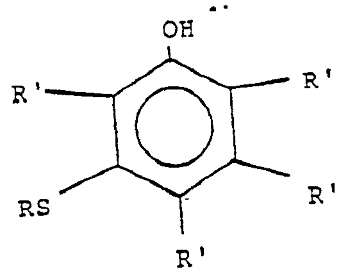

(X)

formules VIII, IX et X dans lesquelles les groupes R' sont des groupes hydrocarbyle identiques ou différents et R est un groupe hydrocarbyle ayant jusqu'à 10 atomes de carbone.

2. Composition suivant la revendication 1, dans laquelle ladite matière organique est une huile minérale.

3. Procédé de préparation d'un hydrocarbylthiophénol suivant la revendication 1, caractérisé en ce qu'il consiste à faire réagir un excès de disulfure d'hydrocarbyle avec une proportion molaire d'un phénol qui est dépourvu de substitution sur les positions devant être substituées par des groupes hydrocarbylthio, en présence de moins d'une proportion molaire d'un phénate d'aluminium catalyseur, à une température de 0 à 300 °C.